# EUROPEAN PATENT APPLICATION

(11) **EP 4 506 404 A1**
(43) Date of publication of application: **12.02.2025**
(21) Application number: 23386075.8
(22) Date of filing: 07.08.2023
(51) Int. Cl.: C08L 5/08, C12N 5/00

(54) **MACROMOLECULAR CROWDING IN CELL CULTURE**

(71) Applicant: University College Dublin, National University of Ireland, Dublin 4 (IE)
(72) Inventor: The designation of the inventor has not yet been filed
(74) Representative: Barker Brettell LLP

(57) **Abstract**

A composition for cell culture, the composition comprising glycosaminoglycan (GAG) having a molecular weight of from 60 to 2000 kDa, wherein the GAG has a molecular weight distribution comprising two or more peaks. Use of the composition for enhancing or influencing ECM deposition in a cell culture; or as a macromolecular crowding agent in cell culture. A cell culture medium comprising the composition. A method of eukaryote cell culture; a method of enhancing or influencing ECM deposition in eukaryote cell culture; and a method of manufacturing a cell culture medium.

## Description

The present invention relates to compositions comprising macromolecular crowding agents for use in cell culture, and associated methods of cell culture.

The intracellular space [1, 2] and the extracellular matrix (ECM) [3, 4] are densely occupied by a high number of unrelated macromolecular species. This packing is referred to as macromolecular crowding (MMC) as no single species is in particular high concentration [5, 6]. Alongside MMC, the term excluded volume effect (EVE) is frequently used that denotes electrostatic interaction- [2], chemical interaction- [8] and steric hindrance- [9] induced mutual volume exclusion of macromolecules within a crowded and structureless medium [10]. MMC, via molecular motion obstruction, affects biochemical equilibria [11], biological processes [12] and macromolecular diffusion [13]. Computational and experimental studies in simple reactant solutions and prokaryotic cell culture systems have comprehensively shown that molecular motion and/or diffusion depend on crowder's physicochemical (e.g. concentration [14], size / molecular weight [15], charge [16], polydispersity [17]) properties. Considering that biological systems are comprised of a diverse range of macromolecules, mixed MMC systems (i.e. cocktails of either different molecules or of different molecular weights of the same molecule), despite their immense complexity, are continuously gaining pace due to their physiological relevance [18-21].

Although in eukaryotic cell culture MMC is used unconsciously (i.e. the term MMC was not used) since 1978 [22] and consciously (i.e. the term MMC was used) since 2007 [23], only a handful of studies have assessed its mode of action. For example, an early work showed the negatively charged dextran sulphate 500 kDa and polysodium-4-styrene sulfonate 200 kDa to result in faster and higher ECM deposition than the negatively charged dextran sulphate 10 kDa and the neutral Ficoll^{®} (FC) 70 kDa and FC 400 kDa [24]. It was then shown that a FC cocktail (70 kDa and 400 kDa) can enhance ECM deposition, but still at lower rate and amount than dextran sulphate 500 kDa [25]. A later study demonstrated carrageenan (CR), due to its negative change and polydispersity, to induce higher ECM deposition than the dextran sulphate 500 kDa and the FC cocktail (70 kDa and 400 kDa) [26]. A subsequent study showed that appropriate cocktails of FC (e.g. 70 kDa, 400 kDa, 1,000 kDa) and dextran sulphate (e.g. 10 kDa, 100 kDa, 500 kDa) can almost match the effectiveness of CR, due to efficient EVE [27].

Although in traditional computational and thermodynamic studies, MMC agents are often assumed inert [28], dextran sulphate has been shown to activate fibrotic pathways [29]; CR has been shown to increase osteogenic and chondrogenic lineage commitment [30-32]; and the FC cocktail (70 kDa and 400 kDa) has been shown to induce adipogenic differentiation [33, 34]. All these data clearly illustrate that the chemistry of the MMC agent is also important in eukaryotic cell cultures and key determinant of cell fate.

Hyaluronic acid (HA) is a negatively charged glycosaminoglycan that despite being used extensively in eukaryotic cell culture, only recently it (1,500 kDa) was assessed as a MMC agent [35]. Although HA has shown little success in enhancing and accelerating ECM deposition, when it (10 kDa, 60 kDa, 100 kDa, 500 kDa, 1,000 kDa) was compared to CR and the FC cocktail (70 kDa and 400 kDa) [36], its chondrogenic [37-39] and osteogenic [40-42] induction capacities have been well-established in the literature. This is particularly important in the development of cell-based therapies for cartilage and bone regeneration, as among the MMC agents assessed to-date only CR can effectively do so [30-32], but its use in clinical setting may be jeopardised by its (questionable [43-45]) association with colitis [46].

Therefore, there is a need to improve the provision of MMC agents for eukaryotic cell culture for enhanced ECM deposition.

According to a first aspect of the invention there is provided a composition for cell culture, the composition comprising glycosaminoglycan (GAG) having a molecular weight of from 60 to 2000 kDa, wherein the GAG has a molecular weight distribution comprising two or more peaks.

According to a second aspect of the invention there is a cell culture medium comprising the composition of the first aspect.

The invention herein has assessed the physicochemical properties (e.g. hydrodynamic radius, zeta potential and polydispersity index) of CR, FC cocktail (70 kDa and 400 kDa) and different concentrations of different molecular weights HAs and appropriate cocktails thereof and their effect in equine bone marrow mesenchymal stromal cell (eBM-MSC) function (e.g. morphology, viability, metabolic activity, proliferation and ECM deposition) and tri-lineage differentiation (e.g. osteogenic, adipogenic and chondrogenic). Advantageously, a combination of glycosaminoglycans (discussed herein as HA cocktails) has been found to have a surprising, beneficial and specific effect in the deposition of specific collagens. In particular, the composition of the invention provides enhanced and specific ECM deposition in cell culture, whereby production of collagen types III, IV, V, and VI is selectively enhanced over collagen I. The combination of glycosaminoglycans according to the invention significantly outperformed the Ficoll^{®} cocktail and carrageenan agents in collagen type IV and collagen type VI deposition; and outperformed carrageenan in laminin deposition.

In one embodiment, the composition or cell culture medium has a polydispersity index of at least 0.4. In one embodiment, the composition or cell culture medium has a polydispersity index of at least 0.5. In one embodiment, the composition or cell culture medium has a polydispersity index of at least 0.6.

The molecular weight may be measured by Dynamic Light Scattering (DLS), for example by a Zetasizer ZS90, Malvern Instruments, UK. The molecular weight may be measured by Dynamic Light Scattering (DLS) as described herein.

In one embodiment, the at least two molecular weight peaks (i.e. the apex of the peaks) are at least 10kDa apart. In particular, the difference between the apex of one peak and the apex of the other peak is at least 10kDa. In another embodiment, the at least two molecular weight peaks (i.e. the apex of the peaks) are at least 20kDa apart. In another embodiment, the at least two molecular weight peaks (i.e. the apex of the peaks) are at least 50kDa apart. In another embodiment, the at least two molecular weight peaks (i.e. the apex of the peaks) are at least 400kDa apart. In another embodiment, the at least two molecular weight peaks (i.e. the apex of the peaks) are at least 500kDa apart.

In one embodiment, the GAG has a molecular weight distribution comprising three or more peaks. In another embodiment, the GAG has a molecular weight distribution comprising four or more peaks. In another embodiment, the GAG has a molecular weight distribution comprising five or more peaks. The GAG molecules represented by at least two separate peaks on a molecular weight distribution may or may not have an overlapping molecular weight range.

The composition or cell culture medium may comprise glycosaminoglycan (GAG) molecules having a molecular weight of between 60 kDa and 2000 kDa, wherein there are at least two ranges of molecular weights of the GAG molecules represented as at least two distinct peaks on a molecular weight distribution chart, wherein the Y-axis is the quantity of GAG molecules and the X-axis is the molecular weight of the GAG molecules ranging from 60kDa to 2000kDa.

In one embodiment, the composition or cell culture medium may comprise GAG molecules having a molecular weight of between 60 kDa and 2000 kDa; wherein 30-70% by weight of the GAGs are 66-99 kDa, and 30-70% by weight are 100-185 kDa. In another embodiment, the composition or cell culture medium may comprise GAG molecules having a molecular weight of between 60 kDa and 2000 kDa; wherein 40-60% by weight of the GAGs are 66-99 kDa, and 40-60% by weight are 100-185 kDa. In another embodiment, the composition or cell culture medium may comprise GAG molecules having a molecular weight of between 60 kDa and 2000 kDa; wherein 50% by weight of the GAGs are 66-99 kDa, and 50% by weight are 100-1 85 kDa.

In one embodiment, the composition or cell culture medium may comprise GAG molecules having a molecular weight of between 60 kDa and 2000 kDa; wherein 30-70% by weight of the GAGs are 66-99 kDa, and 30-70% by weight are 301-465 kDa. In another embodiment, the composition or cell culture medium may comprise GAG molecules having a molecular weight of between 60 kDa and 2000 kDa; wherein 40-60% by weight of the GAGs are 66-99 kDa, and 40-60% by weight are 301-465 kDa. In another embodiment, the composition or cell culture medium may comprise GAG molecules having a molecular weight of between 60 kDa and 2000 kDa; wherein 50% by weight of the GAGs are 66-99 kDa, and 50% by weight are 301-465 kDa.

In one embodiment, the composition or cell culture medium may comprise GAG molecules having a molecular weight of between 60 kDa and 2000 kDa; wherein 30-70% by weight of the GAGs are 66-99 kDa, and 30-70% by weight are 1.01-1.80 MDa. In another embodiment, the composition or cell culture medium may comprise GAG molecules having a molecular weight of between 60 kDa and 2000 kDa; wherein 40-60% by weight of the GAGs are 66-99 kDa, and 40-60% by weight are 1.01-1.80 MDa. In another embodiment, the composition or cell culture medium may comprise GAG molecules having a molecular weight of between 60 kDa and 2000 kDa; wherein 50% by weight of the GAGs are 66-99 kDa, and 50% by weight are 1.01-1.80 MDa.

In one embodiment, the composition or cell culture medium may comprise GAG molecules having a molecular weight of between 60 kDa and 2000 kDa; wherein 30-70% by weight of the GAGs are 100-185 kDa, and 30-70% by weight are 301-465 kDa. In another embodiment, the composition or cell culture medium may comprise GAG molecules having a molecular weight of between 60 kDa and 2000 kDa; wherein 40-60% by weight of the GAGs are 100-185 kDa, and 40-60% by weight are 301-465 kDa. In another embodiment, the composition or cell culture medium may comprise GAG molecules having a molecular weight of between 60 kDa and 2000 kDa; wherein 50% by weight of the GAGs are 100-185 kDa, and 50% by weight are 301-465 kDa.

In one embodiment, the composition or cell culture medium may comprise GAG molecules having a molecular weight of between 60 kDa and 2000 kDa; wherein 30-70% by weight of the GAGs are 100-185 kDa, and 30-70% by weight are 1.01-1.80 MDa. In another embodiment, the composition or cell culture medium may comprise GAG molecules having a molecular weight of between 60 kDa and 2000 kDa; wherein 40-60% by weight of the GAGs are 100-185 kDa, and 40-60% by weight are 1.01-1.80 MDa. In another embodiment, the composition or cell culture medium may comprise GAG molecules having a molecular weight of between 60 kDa and 2000 kDa; wherein 50% by weight of the GAGs are 100-185 kDa, and 50% by weight are 1.01-1.80 MDa.

In one embodiment, the composition or cell culture medium may comprise GAG molecules having a molecular weight of between 60 kDa and 2000 kDa; wherein 30-70% by weight of the GAGs are 301-465 kDa, and 30-70% by weight are 1.01-1.80 MDa. In another embodiment, the composition or cell culture medium may comprise GAG molecules having a molecular weight of between 60 kDa and 2000 kDa; wherein 40-60% by weight of the GAGs are 301-465 kDa, and 40-60% by weight are 1.01-1.80 MDa. In another embodiment, the composition or cell culture medium may comprise GAG molecules having a molecular weight of between 60 kDa and 2000 kDa; wherein 50% by weight of the GAGs are 301-465 kDa, and 50% by weight are 1.01-1.80 MDa.

The GAG may comprise molecular weights in a range of from 66kDa to 99kDa, which may be included in the medium at a concentration of from 5 to 15 mg/mL, and/or molecular weights in the range of from 100kDa to 185kDa, which may be included in the medium at a concentration of from 5 to 15 mg/mL. Preferably the medium comprises GAG molecular weights in a range of from 66kDa to 99kDa at a concentration of from 5 to 15 mg/mL and comprises GAG molecular weights in the range of from 100kDa to 185kDa at a concentration of from 5 to 15 mg/mL.

The GAG may comprise molecular weights in a range of from 66kDa to 99kDa, which may be included in the medium at a concentration of from 5 to 15 mg/mL, and/or molecular weights in the range of from 301 to 465 kDa, which may be included in the medium at a concentration of from 5 to 15 mg/mL. Preferably the medium comprises GAG molecular weights in a range of from 66kDa to 99kDa at a concentration of from 5 to 15 mg/mL and comprises GAG molecular weights in the range of from 301 to 465 kDa at a concentration of from 5 to 15 mg/mL.

The GAG may comprise molecular weights in a range of from 66kDa to 99kDa, which may be included in the medium at a concentration of from 5 to 15 mg/mL, and/or molecular weights in the range of from 1.01 to 1.80 MDa, which may be included in the medium at a concentration of from 5 to 15 mg/mL. Preferably the medium comprises GAG molecular weights in a range of from 66kDa to 99kDa at a concentration of from 5 to 15 mg/mL and comprises GAG molecular weights in the range of from 1.01 to 1.80 MDa at a concentration of from 5 to 15 mg/mL.

The GAG may comprise molecular weights in a range of from 100kDa to 185kDa, which may be included in the medium at a concentration of from 5 to 15 mg/mL, and/or molecular weights in the range of from 301 to 465 kDa, which may be included in the medium at a concentration of from 5 to 15 mg/mL. Preferably the medium comprises GAG molecular weights in a range of from 100kDa to 185kDa at a concentration of from 5 to 15 mg/mL and comprises GAG molecular weights in the range of from 301 to 465 kDa at a concentration of from 5 to 15 mg/mL.

The GAG may comprise molecular weights in a range of from 100kDa to 185kDa, which may be included in the medium at a concentration of from 5 to 15 mg/mL, and/or molecular weights in the range of from 1.01 to 1.80 MDa, which may be included in the medium at a concentration of from 5 to 15 mg/mL. Preferably the medium comprises GAG molecular weights in a range of from 100kDa to 185kDa at a concentration of from 5 to 15 mg/mL and comprises GAG molecular weights in the range of from 1.01 to 1.80 MDa at a concentration of from 5 to 15 mg/mL.

The GAG may comprise molecular weights in a range of from 301 to 465 kDa, which may be included in the medium at a concentration of from 5 to 15 mg/mL, and/or molecular weights in the range of from 1.01 to 1.80 MDa, which may be included in the medium at a concentration of from 5 to 15 mg/mL. Preferably the medium comprises GAG molecular weights in a range of from 301 to 465 kDa at a concentration of from 5 to 15 mg/mL and comprises GAG molecular weights in the range of from 1.01 to 1.80 MDa at a concentration of from 5 to 15 mg/mL.

According to another aspect of the invention there is provided a composition for cell culture, the composition comprising:
- cell culture medium, wherein the cell culture medium comprises:
   GAG molecular weights in a range of from 66kDa to 99kDa at a concentration of from 5 to 15 mg/mL and comprises GAG molecular weights in the range of from 100kDa to 185kDa at a concentration of from 5 to 15 mg/mL;
   GAG molecular weights in a range of from 66kDa to 99kDa at a concentration of from 5 to 15 mg/mL and comprises GAG molecular weights in the range of from 301 to 465 kDa at a concentration of from 5 to 15 mg/mL;
   GAG molecular weights in a range of from 66kDa to 99kDa at a concentration of from 5 to 15 mg/mL and comprises GAG molecular weights in the range of from 1.01 to 1.80 MDa at a concentration of from 5 to 15 mg/mL;
   GAG molecular weights in a range of from 100kDa to 185kDa at a concentration of from 5 to 15 mg/mL and comprises GAG molecular weights in the range of from 301 to 465 kDa at a concentration of from 5 to 15 mg/mL;
   GAG molecular weights in a range of from 100kDa to 185kDa at a concentration of from 5 to 15 mg/mL and comprises GAG molecular weights in the range of from 1.01 to 1.80 MDa at a concentration of from 5 to 15 mg/mL; or
   GAG molecular weights in a range of from 301 to 465 kDa at a concentration of from 5 to 15 mg/mL and comprises GAG molecular weights in the range of from 1.01 to 1.80 MDa at a concentration of from 5 to 15 mg/mL.

According to another aspect of the invention there is provided a composition of glycosaminoglycans for use in cell culture, the composition comprising:
- GAG molecules having a molecular weight from 60 kDa to 100 kDa and GAG molecules having a molecular weight from 300 kDa to 2000 kDa;
- GAG molecules having a molecular weight from 60 kDa to 100 kDa and GAG molecules having a molecular weight from 300 kDa to 500 kDa;
- GAG molecules having a molecular weight from 60 kDa to 100 kDa and GAG molecules having a molecular weight from 1 to 2 MDa;
- GAG molecules having a molecular weight from 100 kDa to 200 kDa and GAG molecules having a molecular weight from 300 KDa to 2 MDa;
- GAG molecules having a molecular weight from 100 kDa to 200 kDa and GAG molecules having a molecular weight from 300 KDa to 500 KDa;
- GAG molecules having a molecular weight from 100 kDa to 200 kDa and GAG molecules having a molecular weight from 1 to 2 MDa; or
- GAG molecules having a molecular weight from 60 kDa to 500 kDa and GAG molecules having a molecular weight from 1 to 2 MDa;
- GAG molecules having a molecular weight from 100 kDa to 500 kDa and GAG molecules having a molecular weight from 1 to 2 MDa; or
- GAG molecules having a molecular weight from 300 kDa to 500 kDa and GAG molecules having a molecular weight from 1 to 2 MDa.

In embodiments of the invention, the use in cell culture may be for macromolecular crowding. The GAG may be a macromolecular crowding agent.

### The glycosaminoglycan

In one embodiment, the GAG is a negatively charged GAG. In a particularly preferred embodiment, the GAG comprises or consists of hyaluronic acid (HA).

In an alternative embodiment, the GAG comprises or consist of dermatan sulphate, chondroitin sulphate, heparin, or heparin sulphate; or combinations thereof. In an alternative embodiment, the GAG comprises or consist of HA, dermatan sulphate, chondroitin sulphate, heparin, or heparin sulphate; or combinations thereof.

The glycosaminoglycan may be sulphated or non-sulphated, such as sulphated HA or non-sulphated HA.

Combinations of the different glycosaminoglycans may be used.

### Cell culture medium

The composition may be a cell culture composition. In one embodiment, the composition is a cell culture medium.

The concentration of glycosaminoglycan in the culture medium may not exceed 20 mg/ml. In another embodiment, the concentration of glycosaminoglycan in the culture medium may not exceed 15 mg/ml. In another embodiment, the concentration of glycosaminoglycan in the culture medium may be from 5 to 20 mg/ml. In another embodiment, the concentration of glycosaminoglycan in the culture medium may be from 10 to 20 mg/ml. In another embodiment, the concentration of glycosaminoglycan in the culture medium may be from 10 to 15 mg/ml.

The cell culture medium may be any suitable medium for the growth and/or maintenance of a cell culture described herein. The medium may comprise one or more components suitable for growth and/or maintenance of a cell culture, such as nutrients, vitamins, minerals, growth factors, antimicrobial, sera, small molecules (i.e. less than 1kDa) and buffer. The cell culture medium may comprise components, such as cytokines, that provide tissue-specific signals for differentiation. The cell culture medium may be tissue specific or not tissue specific. The medium may comprise serum or may be serum free.

The cell culture medium may be a chemically defined medium or a complex medium. The skilled person will be familiar with suitable cell culture medium for cells to be maintained and/or grow. For example, the cell culture medium may comprise basal media, such as MEM (Minimum Essential Medium) or DMEM (Dulbecco's Modified Eagle's Medium), complex medium, such as IMDM (Iscove's Modified Dulbecco's Medium) or RPMI-1640, or serum-free media such as Ham's F-10 or F-12. The basal medium may comprise chemically defined basal media. The cell culture medium may be supplemented with cell feed. The medium may be an osteogenic medium, for example as described herein.

### The Cell Culture

The cells for culture may comprise or consist of eukaryote cells, preferably mammalian cells. The cells may be stem cells, such as mesenchymal stem cells (MSCs). The MSCs may be bone-marrow derived. The cells for culture may comprise or consist of mammalian cells that typically grow in vivo within an extracellular matrix (ECM). In one embodiment, the cells may comprise or consist of equine bone marrow-derived stem cells (eBM-MSC). In another embodiment, the cells may be adipose derived cells, such as adipose derived MSCs. The cells may be any cell type that produces extracellular matrix (ECM).

In one embodiment, the cells for culture may comprise or consist of equine bone marrow derived mesenchymal stromal cells, adipose derived mesenchymal stromal cells, or human bone marrow mesenchymal stromal cells.

In another embodiment, the cells for culture may comprise or consist of dermal fibroblasts, corneal fibroblasts, lung fibroblasts, breast fibroblasts, tendon fibroblasts (tenocytes), bone marrow, adipose derived mesenchymal stromal cells, umbilical cord mesenchymal stromal cells, osteoblasts, or chondrocytes; or combinations thereof.

In another embodiment, the cells for culture may comprise or consist of human dermal fibroblasts, human corneal fibroblasts, human lung fibroblasts, human breast fibroblasts, human or equine tendon fibroblasts (tenocytes), human bone marrow, adipose derived mesenchymal stromal cells. umbilical cord mesenchymal stromal cells, human osteoblasts, or human chondrocytes; or combinations thereof.

In one embodiment, the cells are human cells, such as human MSCs. In one embodiment, the cells are not human embryonic cells.

The cell culture may be *in vitro.*

### Other Aspects

According to another aspect of the invention there is provided a cell culture medium comprising the composition according to the invention herein.

According to another aspect of the invention there is provided a method of eukaryote cell culture, the method comprising the step of incubating eukaryote cells in a cell culture medium according to the invention.

Medium may be changed one or more times during the culture period. Medium may be changed periodically. In one embodiment, medium is changed about every 3-4 days.

The cells may be cultured under conditions suitable for growth or maintenance. In an embodiment wherein the cells are stem cells, the cells may be cultured under conditions suitable for differentiation. Culturing the cells may be at a temperature and atmosphere suitable for growth of the cells, such as standard conditions (e.g. about 37°C and about 5% CO₂ for example for mammalian cells). The method of cell culture may comprise a method of cell differentiation. The method of cell culture may be a method of osteogenesis from MSCs, for example as described herein.

The culture may be a continuous culture or a batch culture, such as a fed-batch culture.

The invention may be used to enhance ECM deposition. Therefore, according to another aspect of the invention there is provided the use of a composition according to the invention for enhancing ECM deposition in a cell culture.

The use of the composition may be to preferentially enhance one or more of collagen III, IV, V or VI deposition in a cell culture. In one embodiment, the use of the composition may be to preferentially enhance collagen III or IV deposition in a cell culture. The use of the composition may be to preferentially enhance collagen III or V deposition in a cell culture relative to collagen I production. The use of the composition may be to enhance collagen deposition in a cell culture, but optionally not collagen I deposition. Additionally or alternatively, the use of the composition may be to preferentially enhance laminin deposition in the cell culture.

According to another aspect of the invention there is provided the use of a composition according to the invention as a macromolecular crowding agent in cell culture.

According to another aspect of the invention there is provided a cell population or tissue produced according to the method of the invention herein.

### Manufacture

According to another aspect of the invention there is provided method of manufacturing the composition according to the invention; the method comprising mixing together two or more populations GAG molecules having different molecular weight ranges, to form the composition according to the invention.

According to another aspect of the invention there is provided method of manufacturing the composition according to the invention; the method comprising mixing together a first population of GAG molecules with at least a second population of GAG molecules, wherein the first population of GAG molecules has a lower average molecular weight than the second population of GAG molecules.

According to another aspect of the invention there is provided method of manufacturing a composition for cell culture,

The method may further comprise addition of the composition to a cell culture medium. Therefore, according to another aspect of the invention there is provided method of manufacturing a cell culture medium, the method comprising adding the composition according to the invention to cell culture medium.

According to another aspect of the invention there is provided method of manufacturing a cell culture medium, the method comprising adding a first population of GAG molecules to a cell culture medium and adding at least a second population of GAG molecules to the cell culture medium, wherein the first population of GAG molecules has a lower average molecular weight than the second population of GAG molecules.

The first and second populations of GAG molecules may be added together, pre-mixed, concurrently, separately or sequentially.

Preferably, the first population of glycosaminoglycan has a different average molecular weight than the second population of glycosaminoglycan. The average molecular weight of the first population of glycosaminoglycan molecules may be at least 400 kDa less than the average molecular weight of the second population of glycosaminoglycan molecules. In another embodiment, the average molecular weight of the first population of glycosaminoglycan molecules may be at least 100 kDa less than the average molecular weight of the second population of glycosaminoglycan molecules. In another embodiment, the average molecular weight of the first population of glycosaminoglycan may be at least 200 kDa less than the average molecular weight of the second population of glycosaminoglycan molecules. In another embodiment, the average molecular weight of the first population of glycosaminoglycan molecules may be at least 400 kDa less than the average molecular weight of the second population of glycosaminoglycan molecules.

The first and second populations of glycosaminoglycan molecules may not have an overlapping molecular weight range.

The first and second populations of glycosaminoglycan may be provided in a ratio of 1:1 w/w (first:second). In one embodiment, the first and second populations of glycosaminoglycan may be provided in a ratio of from 1:4 to 4:1 w/w. In another embodiment, the first and second populations of glycosaminoglycan may be provided in a ratio of from 1:3 to 3:1 w/w. In another embodiment, the first and second populations of glycosaminoglycan may be provided in a ratio of from 1:2 to 2:1 w/w. In another embodiment, the first and second populations of glycosaminoglycan may be provided in a ratio of from 2:1 to 1:1 w/w.

The composition of the invention may have a hydrodynamic radius of 1 nm to 10,000 nm. The composition of the invention may have a neutral to negative charge.

In one embodiment, only two glycosaminoglycan populations are provided (i.e. the first and second). In another embodiment, three, four or more glycosaminoglycan populations may be provided. In one embodiment, there may not be more than two glycosaminoglycan populations, not more than three glycosaminoglycan populations, or not more than four glycosaminoglycan populations.

The first population of GAG molecules may have a molecular weight range from 60 kDa to 100 kDa and the second population of GAG molecules may have a molecular weight range from 100 kDa to 2000 kDa. In another embodiment, the first population of GAG molecules may have a molecular weight range from 60 kDa to 100 kDa and the second population of GAG molecules may have a molecular weight range from 100 kDa to 200 kDa. In another embodiment, the first population of GAG molecules may have a molecular weight range from 66 kDa to 99 kDa and the second population of GAG molecules may have a molecular weight range from 100 kDa to 1.8 MDa. In another embodiment, the first population of GAG molecules may have a molecular weight range from 66 kDa to 99 kDa and the second population of GAG molecules may have a molecular weight range from 100 kDa to 185 KDa.

The first population of GAG molecules may have a molecular weight range from 60 kDa to 100 kDa and the second population of GAG molecules may have a molecular weight range from 300 kDa to 2000 kDa. In another embodiment, the first population of GAG molecules may have a molecular weight range from 66 kDa to 99 kDa and the second population of GAG molecules may have a molecular weight range from 301 kDa to 1.8 MDa.

The first population of GAG molecules may have a molecular weight range from 60 kDa to 100 kDa and the second population of GAG molecules may have a molecular weight range from 300 kDa to 500 kDa. According to another embodiment, the first population of GAG molecules may have a molecular weight range from 66 kDa to 99 kDa and the second population of GAG molecules may have a molecular weight range from 301 kDa to 465 kDa.

The first population of GAG molecules may have a molecular weight range from 60 kDa to 100 kDa and the second population of GAG molecules may have a molecular weight range from 1 to 2 MDa. According to another embodiment, the first population of GAG molecules may have a molecular weight range from 66 kDa to 99 kDa and the second population of GAG molecules may have a molecular weight range from 1 to 1.8 MDa.

The first population of GAG molecules may have a molecular weight range from 100 kDa to 200 kDa and the second population of GAG molecules may have a molecular weight range from 300 KDa to 2 MDa. According to another embodiment, the first population of GAG molecules may have a molecular weight range from 100 kDa to 185 kDa and the second population of GAG molecules may have a molecular weight range from 301 KDa to 1.8 MDa.

The first population of GAG molecules may have a molecular weight range from 100 kDa to 200 kDa and the second population of GAG molecules may have a molecular weight range from 300 KDa to 500 KDa. According to another embodiment, the first population of GAG molecules may have a molecular weight range from 100 kDa to 185 kDa and the second population of GAG molecules may have a molecular weight range from 301 KDa to 465 KDa.

The first population of GAG molecules may have a molecular weight range from 100 kDa to 200 kDa and the second population of GAG molecules may have a molecular weight range from 1 to 2 MDa. According to another embodiment, the first population of GAG molecules may have a molecular weight range from 100 kDa to 185 kDa and the second population of GAG molecules may have a molecular weight range from 1 to 1.8 MDa.

The first population of GAG molecules may have a molecular weight range from 60 kDa to 500 kDa and the second population of GAG molecules may have a molecular weight range from 1 to 2 MDa. According to another embodiment, the first population of GAG molecules may have a molecular weight range from 66 kDa to 465 kDa and the second population of GAG molecules may have a molecular weight range from 1 to 1.8 MDa.

The first population of GAG molecules may have a molecular weight range from 100 kDa to 500 kDa and the second population of GAG molecules may have a molecular weight range from 1 to 2 MDa. According to another embodiment, the first population of GAG molecules may have a molecular weight range from 100 kDa to 465 kDa and the second population of GAG molecules may have a molecular weight range from 1 to 1.8 MDa.

The first population of GAG molecules may have a molecular weight range from 300 kDa to 500 kDa and the second population of GAG molecules may have a molecular weight range from 1 to 2 MDa. According to another embodiment, the first population of GAG molecules may have a molecular weight range from 301 kDa to 465 kDa and the second population of GAG molecules may have a molecular weight range from 1 to 1.8 MDa.

The first and/or second population of GAG molecules may be added at a concentration of from 5 to 15 mg/mL. According to another embodiment, the first and/or second population of GAG molecules may be added at a concentration of from 10 to 15 mg/mL.

According to another embodiment, the first and/or second population of GAG molecules may be added at a concentration of from 5 to 10 mg/mL. According to another embodiment, the first and/or second population of GAG molecules may be added at a concentration of about 10 mg/mL.

The first population of glycosaminoglycan may be provided in the culture medium at a concentration of from 3 to 15 mg/ml. In another embodiment, the first population of glycosaminoglycan may be provided in the culture medium at a concentration of from 5 to 15 mg/ml. In another embodiment, the first population of glycosaminoglycan may be provided in the culture medium at a concentration of from 3 to 10 mg/ml. In another embodiment, the first population of glycosaminoglycan may be provided in the culture medium at a concentration of from 5 to 10 mg/ml.

The second population of glycosaminoglycan may be provided in the culture medium at a concentration of from 3 to 15 mg/ml. In another embodiment, the second population of glycosaminoglycan may be provided in the culture medium at a concentration of from 3 to 12 mg/ml. In another embodiment, the second population of glycosaminoglycan may be provided in the culture medium at a concentration of from 3 to 10 mg/ml. In another embodiment, the second population of glycosaminoglycan may be provided in the culture medium at a concentration of from 5 to 10 mg/ml. In another embodiment, the second population of glycosaminoglycan may be provided in the culture medium at a concentration of from 3 to 8 mg/ml.

The first population of glycosaminoglycan may be provided in the culture medium at a concentration of from 3 to 15 mg/ml and the second population of glycosaminoglycan may be provided in the culture medium at a concentration of from 3 to 15 mg/ml. In another embodiment, the first population of glycosaminoglycan may be provided in the culture medium at a concentration of from 5 to 10 mg/ml and the second population of glycosaminoglycan may be provided in the culture medium at a concentration of from 5 to 10 mg/ml.

In another embodiment, the first population of glycosaminoglycan molecule may have a molecular weight of from 60 kDa to 100 kDa; and the second population of glycosaminoglycan may have a molecular weight of from 100 kDa to 1000 kDa, wherein the first population of glycosaminoglycan is provided in the culture medium at a concentration of from 5 to 10 mg/ml and the second population of glycosaminoglycan is provided in the culture medium at a concentration of from 5 to 10 mg/ml. In another embodiment, the first population of glycosaminoglycan molecule may have a molecular weight of from 60 kDa to 200 kDa; and the second population of glycosaminoglycan may have a molecular weight of from 100 kDa to 1000 kDa, wherein the first population of glycosaminoglycan is provided in the culture medium at a concentration of from 5 to 10 mg/ml and the second population of glycosaminoglycan is provided in the culture medium at a concentration of from 5 to 10 mg/ml.

In another embodiment, the first population of glycosaminoglycan molecule may have a molecular weight of from 100 kDa to 500 kDa; and the second population of glycosaminoglycan may have a molecular weight of from 100 kDa to 1000 kDa, wherein the first population of glycosaminoglycan is provided in the culture medium at a concentration of from 5 to 10 mg/ml and the second population of glycosaminoglycan is provided in the culture medium at a concentration of from 5 to 10 mg/ml.

In another embodiment, the first population of glycosaminoglycan molecule may have a molecular weight of from 60 kDa to 500 kDa; and the second population of glycosaminoglycan may have a molecular weight of from 100 kDa to 500 kDa, wherein the first population of glycosaminoglycan is provided in the culture medium at a concentration of from 5 to 10 mg/ml and the second population of glycosaminoglycan is provided in the culture medium at a concentration of from 5 to 10 mg/ml.

In another embodiment, the first population of glycosaminoglycan molecule may have a molecular weight of from 60 kDa to 500 kDa; and the second population of glycosaminoglycan may have a molecular weight of from 500 kDa to 1000 kDa, wherein the first population of glycosaminoglycan is provided in the culture medium at a concentration of from 5 to 10 mg/ml and the second population of glycosaminoglycan is provided in the culture medium at a concentration of from 5 to 10 mg/ml.

In another embodiment, the first population of glycosaminoglycan molecule may have a molecular weight of about 60-100 kDa; and the second population of glycosaminoglycan may have a molecular weight of about 100-500 kDa, wherein the first population of glycosaminoglycan is provided in the culture medium at a concentration of from 5 to 10 mg/ml and the second population of glycosaminoglycan is provided in the culture medium at a concentration of from 5 to 10 mg/ml.

It is understood that "chemically defined medium" is a growth medium suitable for the *in vitro* cell culture of human or animal cells in which all of the chemical components are known.

The skilled man will appreciate that preferred features of any one embodiment and/or aspect of the invention may be applied to all other embodiments and/or aspects of the invention.

Examples embodying an aspect of the invention will now be described with reference to the following figures:
**Figure 1****:** Hydrodynamic radius (A), zeta potential (B) and polydispersity index (C) analyses of FC and CR (control, CTRL, macromolecules) and HA macromolecules (cocktails). & indicates significantly (p < 0.05) higher to CR and FC. * indicates highest value within a given group. # indicates lowest (p < 0.05) population(s). + indicates highest (p < 0.05) population(s).
**Figure 2****:** SDS-PAGE and complementary densitometry analyses of no macromolecular crowding (- MMC), FC, CR and single HA macromolecules (A: HA 60 kDa, B: HA 100 kDa, C: HA 500 kDa, D: HA 1,000 kDa) in eBM-MSC cultures at day (D) 4 and 8. * indicates significantly (p < 0.05) higher deposition than the - MMC group at a given time point. + indicates highest (p < 0.05) deposition among all groups at a given time point.
**Figure 3****:** SDS-PAGE and complementary densitometry analyses of no macromolecular crowding (- MMC), FC, CR, single HA macromolecules and HA cocktails in eBM-MSC cultures at day (D) 4, 6 and 8. * indicates significantly (p < 0.05) higher deposition than the - MMC group at a given time point. + indicates highest (p < 0.05) deposition among all groups at a given time point.

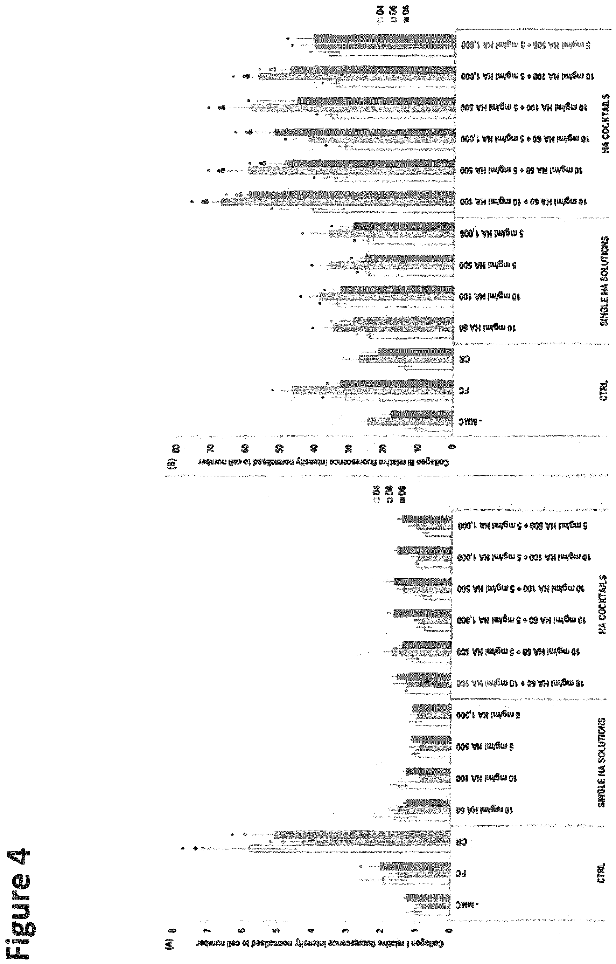

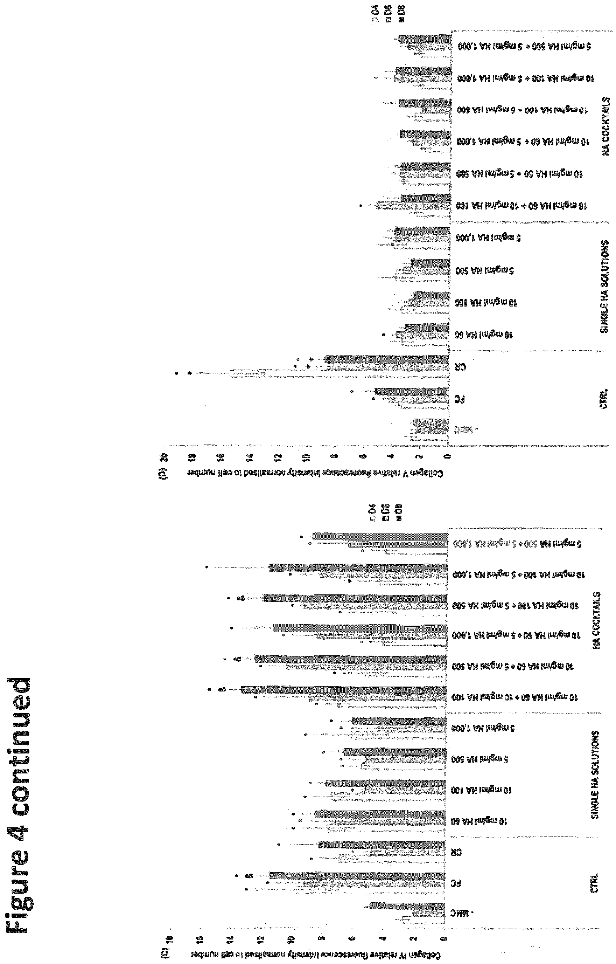

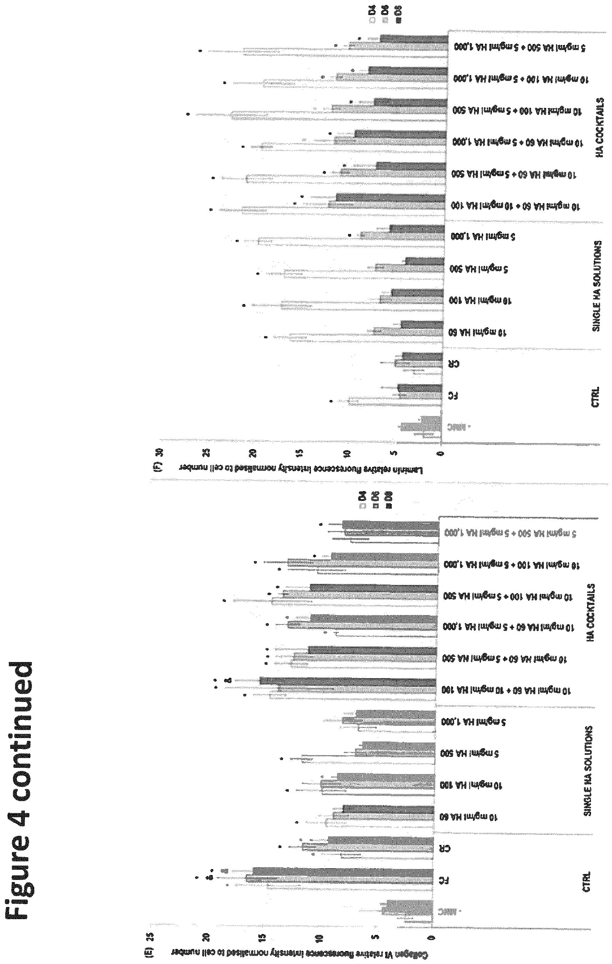

**Figure 4****:** Image fluorescence intensity analysis of no macromolecular crowding (- MMC), FC and CR (control, CTRL, groups) and single HA macromolecules and HA cocktails for collagen type I (A), collagen type III (B), collagen type IV (C), collagen type V (D), collagen type VI (E) and laminin (F) in eBM-MSC cultures at day (D) 4, 6 and 8. * indicates significantly (p < 0.05) higher deposition than - MMC group at a given time point. & indicates significantly (p < 0.05) higher deposition than the single HA molecules at a given time point. + indicates highest (p < 0.05) deposition among all groups at a given time point.
**Figure 5****:** Alizarin red staining (A) and absorbance (B) without macromolecular crowding (- MMC) and with FC, CR, single HA macromolecules and HA cocktails in normal (- induction) and osteogenic (+ induction) media in eBM-MSC cultures at day 14. Scale bars: 100 µm. * indicates significantly (p < 0.05) higher than the respective - induction group. & indicates significantly (p < 0.05) higher than the + induction - MMC group.
**Figure 6****:** Oil red O staining (A) and absorbance (B) without macromolecular crowding (- MMC) and with FC, CR, single HA macromolecules and HA cocktails in normal (- induction) and adipogenic (+ induction) media in eBM-MSC cultures at day 18. Scale bars: 100 µm. * indicates significantly (p < 0.05) higher than the respective - induction group. + indicates highest (p < 0.05) population.
**Figure 7****:** Alcian blue staining (A) and % positive Alcian blue stain (B) without macromolecular crowding (- MMC) and with FC, CR, single HA macromolecules and HA cocktails in normal (- induction) and chondrogenic (+ induction) media in eBM-MSC cultures at day 21. Scale bars: 400 µm. * indicates significantly (p < 0.05) higher than the respective - induction group. & indicates significantly (p < 0.05) higher than the + induction - MMC group.
**Figure 8****:** Tri-lineage assessment of the eBM-MSCs used in the study. Scale bars: 100 µm (for osteogenesis and adipogenesis) and 400 µm (for chondrogenesis). N = 3.
**Figure 9****:** FACS analysis of the eBM-MSCs used in the study. N = 3.
**Figure 10****:** Immunocytochemistry validation of the antibodies used for FACS analysis in eBM-MSC cultures. Scale bars: 100 µm. N = 3.
**Figure 11****:** Bright field microscopy analysis of no macromolecular crowding (-MMC), FC, CR and single HA macromolecules in eBM-MSC cultures at day (D) 4 and 8. Scale bars: 100 µm. N = 3.
**Figure 12****:** Immunocytochemistry analysis for collagen type III (green) of no macromolecular crowding (- MMC), FC, CR and single HA macromolecules in eBM-MSC cultures at day (D) 4 and 8. Scale bars: 100 µm. Blue: DAPI. N = 3.
**Figure 13****:** Image fluorescence intensity analysis for collagen type III of no macromolecular crowding (- MMC), FC, CR and single HA macromolecules in eBM-MSC cultures at day (D) 4 and 8. * indicates significantly (p < 0.05) higher deposition than the - MMC) group at a given time point. + indicates highest (p < 0.05) deposition within a given MMC condition at a given time point. N = 3.
**Figure 14****:** Bright field microscopy analysis of no macromolecular crowding (-MMC), FC, CR, single HA macromolecules and HA cocktails in eBM-MSC cultures at day (D) 4, 6 and 8. Scale bars: 100 µm. N = 3.
**Figure 15****:** Viability analysis of dimethyl sulfoxide (DMSO), no macromolecular crowding (- MMC), FC, CR, single HA macromolecules and HA cocktails in eBM-MSC cultures at day (D) 4, 6 and 8. Scale bars: 50 µm. Green: live cells. Red: dead cells. N = 3.
**Figure 16****:** Viability quantification of no macromolecular crowding (- MMC), FC, CR, single HA macromolecules and HA cocktails in eBM-MSC cultures at day (D) 4, 6 and 8. N = 3.
**Figure 17****:** Proliferation analysis of no macromolecular crowding (- MMC), FC, CR, single HA macromolecules and HA cocktails in eBM-MSC cultures at day (D) 4, 6 and 8. * indicates significantly (p < 0.05) lower proliferation than the - MMC group at a given time point. N = 3.
**Figure 18****:** Metabolic activity analysis of no macromolecular crowding (- MMC), FC, CR, single HA macromolecules and HA cocktails in eBM-MSC cultures at day (D) 4, 6 and 8. * indicates significantly (p < 0.05) higher than the - MMC group at a given time point. N = 3.
**Figure 19****:** Immunocytochemistry analysis for collagen type I (red) of no macromolecular crowding (- MMC), FC, CR, single HA macromolecules and HA cocktails in eBM-MSC cultures at day (D) 4, 6 and 8. DAPI: blue. Scale bars: 100 µm. N = 3.
**Figure 20****:** Immunocytochemistry of collagen type III (green) of no macromolecular crowding (- MMC), FC, CR, single HA macromolecules and HA cocktails in eBM-MSC cultures at day (D) 4, 6 and 8. DAPI: blue. Scale bars: 100 µm. N = 3.
**Figure 21****:** Immunocytochemistry of collagen type IV (green) of no macromolecular crowding (- MMC), FC, CR, single HA macromolecules and HA cocktails in eBM-MSC cultures at day (D) 4, 6 and 8. DAPI: blue. Scale bars: 100 µm. N = 3.
**Figure 22****:** Immunocytochemistry of collagen type V (green) of no macromolecular crowding (- MMC), FC, CR, single HA macromolecules and HA cocktails in eBM-MSC cultures at day (D) 4, 6 and 8. DAPI: blue. Scale bars: 100 µm. N = 3.
**Figure 23****:** Immunocytochemistry of collagen type VI (green) of no macromolecular crowding (- MMC), FC, CR, single HA macromolecules and HA cocktails in eBM-MSC cultures at day (D) 4, 6 and 8. DAPI: blue. Scale bars: 100 µm. N = 3.
**Figure 24****:** Immunocytochemistry of laminin (green) of no macromolecular crowding (- MMC), FC, CR, single HA macromolecules and HA cocktails in eBM-MSC cultures at day (D) 4, 6 and 8. DAPI: blue. Scale bars: 100 µm. N = 3.

### Example 1 - Macromolecular crowding in equine bone marrow mesenchymal stromal cell cultures using single and double hyaluronic acid macromolecules.

### Summary

Macromolecular crowding (MMC) enhances and accelerates extracellular matrix (ECM) deposition in eukaryotic cell culture. Single hyaluronic acid (HA) molecules have not induced a notable increase in the amount and rate of deposited ECM. Thus, herein we assessed the physicochemical properties and biological consequences in equine bone marrow mesenchymal stromal cell cultures of single and mixed HA molecules and correlated them to the most widely used MMC agents, the Ficoll^{®} cocktail (FC) and carrageenan (CR). Dynamic light scattering analysis revealed that all HA cocktails had significantly higher hydrodynamic radius than the FC and CR; the FC and the 0.5 mg/ml 100 kDa and 500 kDa single HA molecules had the highest charge; and, in general, all molecules had high polydispersity index. Biological analyses revealed that none of the MMC agents affected cell morphology and basic cell functions; in general, CR outperformed all other macromolecules in collagen type I and V deposition; FC, the individual HA molecules and the HA cocktails outperformed CR in collagen type III deposition; FC outperformed CR and the individual HA molecules and the HA cocktails outperformed their constituent HA molecules in collagen type IV deposition; FC and certain HA cocktails outperformed CR and constituent HA molecules in collagen type VI deposition; and all individual HA molecules outperformed FC and CR and the HA cocktails outperformed their constituent HA molecules in laminin deposition. With respect to tri-lineage analysis, CR and HA enhanced chondrogenesis and osteogenesis, whilst FC enhanced adipogenesis. This work opens new avenues in mixed MMC in eukaryotic cell culture.

### 1. Materials and methods

### 1.1. Materials

Cell culture plasticware and chemicals / reagents were purchased from Sarstedt (Germany) and Sigma Aldrich (Greece), respectively, unless otherwise stated (catalogue numbers are mentioned in brackets). HAs of different molecular weights [60 kDa (66 kDa to 99 kDa), 100 kDa (100 kDa to 150 kDa), 500 kDa (301 kDa to 450 kDa) and 1,000 kDa (750 kDa to 1,000 kDa)] were purchased from Lifecore^{™} Biomedical (USA). eBM-MSCs were provided by Dr Jayesh Dudhia and Prof Roger Smith, Royal Veterinary College, UK.

### 1.2. Dynamic light scattering (DLS) analysis

Hydrodynamic radius, zeta potential and polydispersity index were assessed using a Zetasizer ZS90, Malvern Instruments, UK. The macromolecules were dissolved phosphate buffered saline (PBS).

### 7.J. Cell culture

eBM-MSCs were expanded until passage 4 in Dulbecco's Modified Eagle Medium high glucose (DMEM-HG, D6429), 10 % foetal bovine serum (FBS, F7524) and 1 % penicillin / streptomycin (P/S, Biosera XC-A4122) at 37 °C and 5 % CO₂ humidified atmosphere. At passage 5, cells were seeded at 15,000 cells/cm² density in DMEM-HG, 10 % FBS, 1 % P/S and 100 µM L-ascorbic acid 2-phosphate sesquimagnesium salt hydrate (A8960). After 24 h, media were changed and replaced with media containing DMEM-HG, 10 % FBS, 1 % P/S, 100 µM L-ascorbic acid 2-phosphate sesquimagnesium salt hydrate and 75 µg/ml CR (C1013); or 37.5 mg/ml FC 70 KDa (F2878) and 25 mg/ml FC 400 KDa (F4375); or different concentrations of different molecular weights HAs; or appropriate cocktails of HAs. The concentration range of the single HA molecular weights (0.5, 1, 5 and 10 mg/ml for the 60, 100 and 500 kDa HA and 0.1, 0.5, 1 and 5 mg/ml for the 1,000 kDa HA) was informed from our previous work with equine adipose derived MSCs [36]. The concentrations of the HA cocktails were based on data derived from the single HA molecules analyses. Media without MMC were used as control. All MMC agents were sterilised under UV light for 15 min and solubilised in DMEM-HG, 10 % FBS, I % P/S, 100 µM L-ascorbic acid 2-phosphate sesquimagnesium salt hydrate at 37 °C until no particles in suspension were observed. Media were changed every 2 days until day 8.

### 1.4. Tri-lineage differentiation analysis

Tri-lineage analysis was conducted as has been described in detailed previously [36]. For osteogenesis, 5,000 cells/cm² were seeded in 48-well plates and osteogenesis was induced with 100 nM dexamethasone (D4902), 100 µM L-ascorbic acid 2-phosphate sesquimagnesium salt hydrate (A8960), 10 mM β-glycerophosphate disodium salt hydrate (G9422), 10 % FBS and 1 % P/S with or without MMC supplementation. Media were changed every 3 days and at day 14, cell layers were treated with methanol at 4 °C for 20 min and calcium deposits were detected by adding 400 µl of 40 mM alizarin red S stain (A5533), pH 4.1. After visualisation with an inverted microscope (Leica Microsystems, Germany), semi-quantification analysis was performed by alizarin red extraction from calcium deposits, using standard acetic acid, heating, centrifugation and ammonium hydroxide steps. Absorbance was read at 405 nm using a microplate reader (Varioskan Flash, ThermoFisher Scientific, Ireland). For adipogenesis, 2,500 cells/cm² were seeded in 48-well plates and adipogenesis was induced with 0.1 µM dexamethasone, 500 µM 3-isobutyl-1-methylxanthine (15879), 200 µM indomethacin (17378), 15 % horse serum (H1270) and 1 % P/S with or without MMC supplementation. Media were changed every 3 days and at day 18, cell layers were treated with 10 % formalin for 15 min at room temperature and 400 µl of 0.3 % red oil O stain (O1391) in 60 % isopropanol. Lipid droplets were visualised with an inverted microscope (Leica Microsystems, Germany) and then semi-quantification analysis was performed by adding 100 % isopropanol for 10 min and reading absorbance at 540 nm using a microplate reader (Varioskan Flash, ThermoFisher Scientific, Ireland). For chondrogenesis, pellet culture was used in round bottom 96-well plates by growing 500,000 cells for 48 h in 200 µl of chondrogenic media [serum-free DMEM, 1 % P/S, 100 nM dexamethasone, 1TS+1 liquid media supplement 100x (I2521), 40 µg/ml L-proline (P5607), 50 µg/ml L-ascorbic acid 2-phosphate sesquimagnesium salt hydrate (A8960), 10 ng/ml transforming growth factor β3 (R&D 8420-B3/CF)] and then changing the media to with or without MMC media. Media were changed every 3 days and at day 21, pellets were fixed with 4 % paraformaldehyde (PFA); cryoprotected with increasing gradient (15 and 30 %) sucrose solutions; cut in 5 µm thick sections with a Leica CM1850 (Leica Microsystems, UK) cryotome; stained with Alcian blue (B8438); washed with 1 % acetic acid; stained with 0.1 % nuclear fast red aluminium solution (100121); washed with double-distilled water; dehydrated in 100 % ethanol; mounted using DPX (06522); and imaged with an Olympus BX51 microscope (Olympus Corporation, Tokyo, Japan). Histological analysis was performed with ImageJ (NIH, USA) to calculate the % of positive Alcian blue stained area [47].

### 7.J. Fluorescence activated cell sorting (FACS) analysis

A FACScalibur^{™} (BD, UK) device was used for cell surface markers analysis, as has been described in detail previously [36]. Briefly, at passage 5, cells were trypsinised; suspended at 2 x 10⁶ cells/ml in 2 mM ethylenediaminetetraacetic acid / PBS solution; and 50 µl aliquots were incubated for 30 min at 4 °C with AlexaFluor^{®} 488 anti-human CD29 (BioLegend^{®}, USA, clone TS2/16) [48, 49], FITC mouse anti-human CD34 (BD Pharmingen^{™}, UK, clone 581) [50-52], FITC mouse anti-horse CD44 (Bio-Rad Laboratories, UK, clone CVS8) [50-52], PE mouse anti-human CD90 (BD Pharmingen^{™}, UK, clone 5E10) [48, 49, 53] and PE mouse anti-human CD105 (ThermoFisher Scientific, UK, clone SN6) [51]. PE- and FITC- conjugated mouse IgG1, κ isotype controls (BD Pharmingen^{™}, UK, clone MOPC21) were used to discard unspecific binding and cells alone were used as negative controls to eliminate autofluorescence. SYTOX^{™} Red Dead Cell Stain (ThermoFisher Scientific, UK, S3459) at a final concentration of 1 µM was used to distinguish viable and non-viable cells. Analysis was performed by using BD CellQuest^{™} software (BD, UK). Immunocytochemistry (see below sample preparation) was also carried out to validate the antibodies used for FACS analysis.

### 1.6. Cell morphology, viability, proliferation and metabolic activity analyses

Bright field microscopy (IX73, Olympus Corporation, Japan) was used to assess cell morphology. Live/Dead^{®} viability kit (Invitrogen^{™}, UK, L3224) was used to assess cell viability, as per manufacturer's protocol and samples were visualised using an inverted IX73 Olympus microscope (Olympus Corporation, Japan). 5 regions of interest (ROI) per image were selected; live / dead cells were counted with ImageJ (NIH, USA); and % cell viability was calculated as live cell number against total cell number. Cell proliferation was assessed via nuclei counting, using 4',6-diamidino-2-phenylindole (DAPI, Invitrogen^{™}, UK, D1306) staining and an IX73 inverted Olympus microscope (Olympus Corporation, Japan) for visualisation. 5 ROI per image were selected and cell nuclei were counted with ImageJ software, utilising the watershed plugin to separate overlapped nuclei. Cell metabolic activity was assessed using the alamarBlue^{™} (Invitrogen^{™}, UK, DAL1025) assay, as per manufacturer's protocol. Absorbance at 550 nm and 595 nm excitation and emission, respectively, was measured using an ELx800 (Biotek, UK) plate reader and % reduction of the alamarBlue^{®} dye was calculated with data normalised to the without MMC group.

### 7.7. Gel electrophoresis analysis

At each time point collagen extraction from the cell layers and sodium dodecyl sulphate-polyacrylamide gel electrophoresis (SDS-PAGE) was performed [54]. Briefly, cell layers were washed twice with Hanks' Balanced Salt Solution (HBSS) at 37 °C; digested with porcine gastric mucosa pepsin (P6887; 150 µl per well of a 24-well plate at a final concentration of 0.1 mg/ml in 0.05 M acetic acid) for 2 h at 37 °C under continuous shaking; and then 100 µl of the acidic solution were neutralised with 20 µl of 0.1 N NaOH. Samples were diluted with distilled water and 5 x sample buffer; heated at 95 °C for 5 min; and loaded 3 % stacking and 5 % separation gel. As standard, 15 µl of 0.1 mg/ml in 0.05 M acetic acid bovine skin collagen type 1 (Symatese Biomateriaux, France, CBPE2US010) were used. A Mini-PROTEAN^{®} Tetra Cell electrophoresis system (Bio-Rad Laboratories, UK) was used, first at 50 mV for 30-40 min and then at 110 mV for 50-70 min. The gels were stained with the PlusOne^{™} Silver Staining Kit, Protein (GE Healthcare, UK, 17115001), according to the manufacturer's protocol and to quantify collagen type I deposition, the relative densities of collagen α1(1) and α2(I) chains were evaluated using the background subtraction plugin of ImageJ (NIH, USA) and correlating them to the respective densities of the standard.

### 1.8. Immunocytochemistry analysis

At each time point, cell layers were washed with HBSS; fixed for 15 min with 2 % PFA at 4 °C; blocked with 3 % bovine serum albumin (A7638) solution at room temperature for 30 min; and incubated for 90 min at room temperature with the following primary antibodies: mouse monoclonal anti-collagen type I (ab90395, 1:500 dilution), rabbit polyclonal anti-collagen type III (ab7778, 1:200 dilution), rabbit polyclonal anti-collagen type IV (ab6586, 1:200 dilution), rabbit polyclonal anti-collagen type V (ab7046, 1:200 dilution), rabbit polyclonal anti-collagen type VI (ab6588, 1:200 dilution) and rabbit polyclonal anti-laminin (ab11575, 1:200 dilution); all Abeam, Ireland. The samples were then washed with PBS and incubated for 30 min with the following secondary antibodies at 1:500 dilution: goat anti-rabbit conjugated to AlexaFluor^{®} 488 (A-32731) and goat anti-mouse conjugated AlexaFluor^{®} 555 (A-32727); both ThermoFisher Scientific (Ireland). As negative controls to subtract the background, samples were incubated in PBS without primary antibodies and then with the corresponding secondary antibodies. Samples were also incubated for 5 min with DAPI at 0.5 µg/ml concentration in methanol; mounted with 10 µl of VECTASHIELD^{®} HardSet^{™} Antifade Mounting Medium (H 1500, Vector Laboratories, UK); covered with 8 mm in diameter glass coverslips; and imaged with an IX73 microscope (Olympus Corporation, Japan). The Q-Capture Pro 7 software (Qlmaging, USA) was used for image acquisition. 5 ROI per image were selected and fluorescence intensity was calculated using the ImageJ software (NIH, USA), subtracting from every ROI the average fluorescence intensity value of the negative control. The values were normalised to the corresponding cell number of each ROI.

### 1.9. Statistical analysis

Numerical data are expressed as mean ± standard deviation. Data were processed in Microsoft^{®} Excel and statistical analysis was performed using SigmaPlot^{®} 12.0 software (Systat Software, UK). One way analysis of variance (ANOVA) followed by pairwise multiple comparison (Holm-Sidak's method) was used when the distributions of the populations were normal (Shapiro-Wilk normality test) and the variances of populations were equal (Levene's test). Non-parametric analysis was performed using Kruskal-Wallis followed by Dunn's multiple comparison post-hoc analysis for pairwise multiple comparisons, when the assumptions of parametric analysis were violated. Statistical significance was accepted at *p* < 0.05.

### 2. Hyaluronic Acid

The HA may be supplied in the form of sodium hyaluronate in accordance with the following weight ranges of Table 1. Reference in the Examples to 60kDa HA refers to HA60K, reference to 100kDa HA refers to HA100K, reference to 500kDa HA refers to HA500K and reference to 1000kDa HA refers to HA15M. Therefore, whilst the statements of invention may refer to a molecular weight, such as 60 kDa HA, the population of HA molecules in the batch may vary. Therefore, the stated MW may be the average MW in a population, or it may refer to the following ranges:
60 kDa may be equal to a population consisting of 66-99kDa molecules;
100kDa may be equal to a population consisting of 100-185 kDa molecules;
500 kDa may be equal to a population consisting of 301-465 kDa molecules; and
1000 kDa may be equal to a population consisting of 1.01 to 1.80 MDa molecules.

**Table 1.**

| Molecular Weight Range (Daltons) | 1 Gram Part # |
|---|---|
| <10K | HA5K-1 |
| 10K-20K | HA10K-1 |
| 21K-40K | HA2OK-1 |
| 41K-6SK | HA40K-1 |
| 66K-99K | HA60K-1 |
| 100K-185K | HA100K-1 |
| 151K-300K | HA200K-1 |
| 301K-475K | HA500K-1 |
| 500K-749K | HA700K-1 |
| 750K-1.0M | HA1M-1 |
| 1.01M-1.80M | HA15M-1 |

The composition or cell culture medium of the invention may comprise any combination of two or more HA weight range populations provided in Table 1, optionally not including any of HA5K-1, HA10K-1, HA20K-1 and HA40K-1.

### 3. Results

### 3.1. Cell multipotency analysis

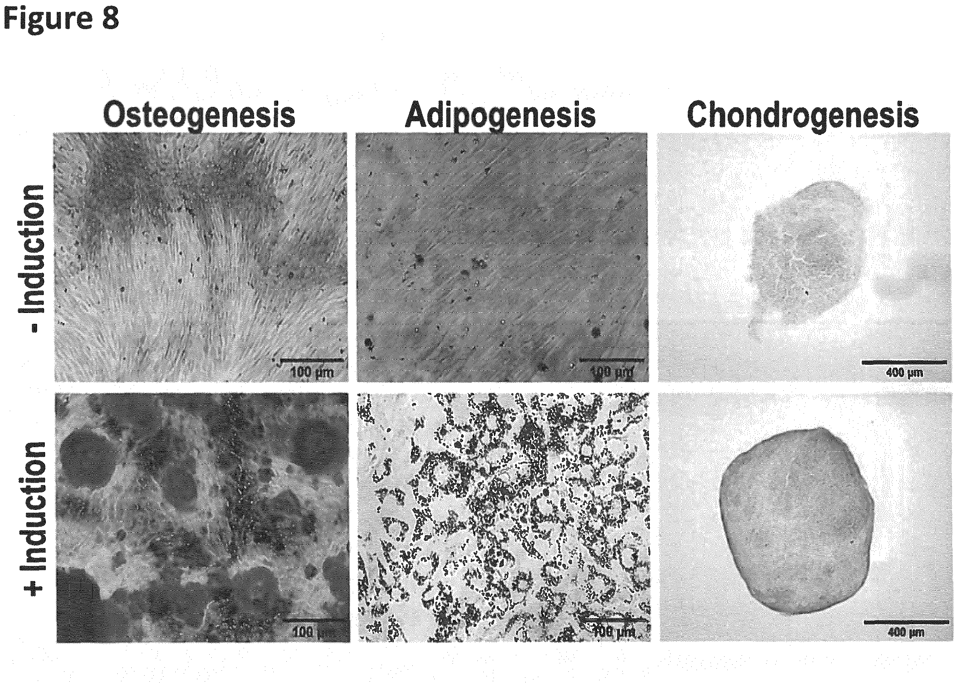

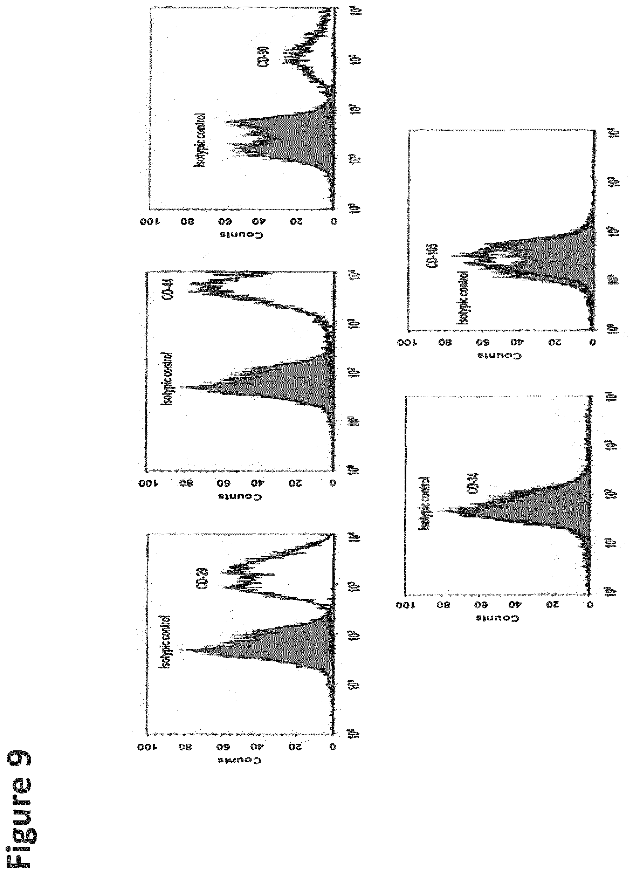

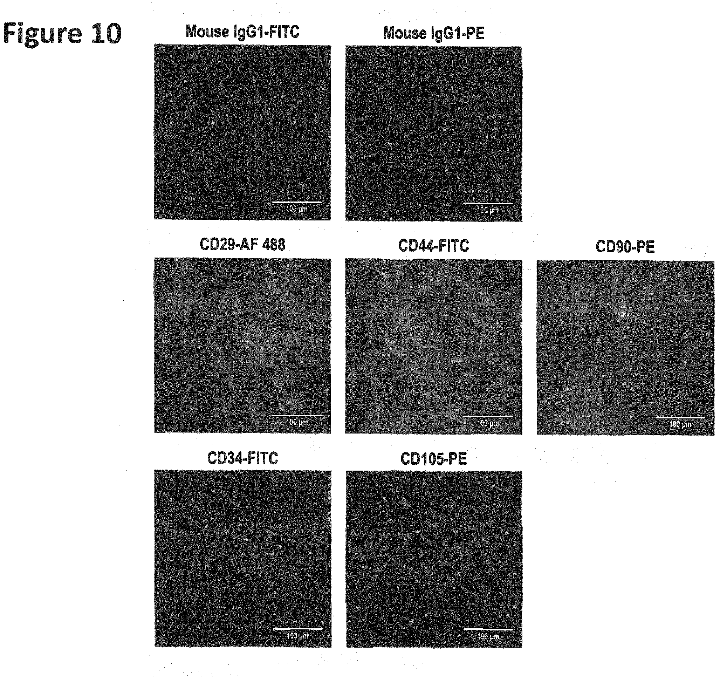

The multipotency of the cells was confirmed via successful osteogenic, adipogenic and chondrogenic induction in respective media **(****Figure 8****).** FACS analysis showed that the cells were positive for CD29 (98.59 % ± 0.59 %), CD44 (85.40 % ± 1.84 %) and CD90 (99.96 % ± 0.02 %) and negative for CD34 (0.41 % ± 0.34 %) and CD105 (0.19 % ± 0.03 %) **(****Figure 9****).** Qualitative immunocytochemistry analysis also confirmed that the cells were positive for CD29, CD44 and CD90 and negative for CD34 and CD105 **(****Figure 10****).**

### 3.2. DLS analysis

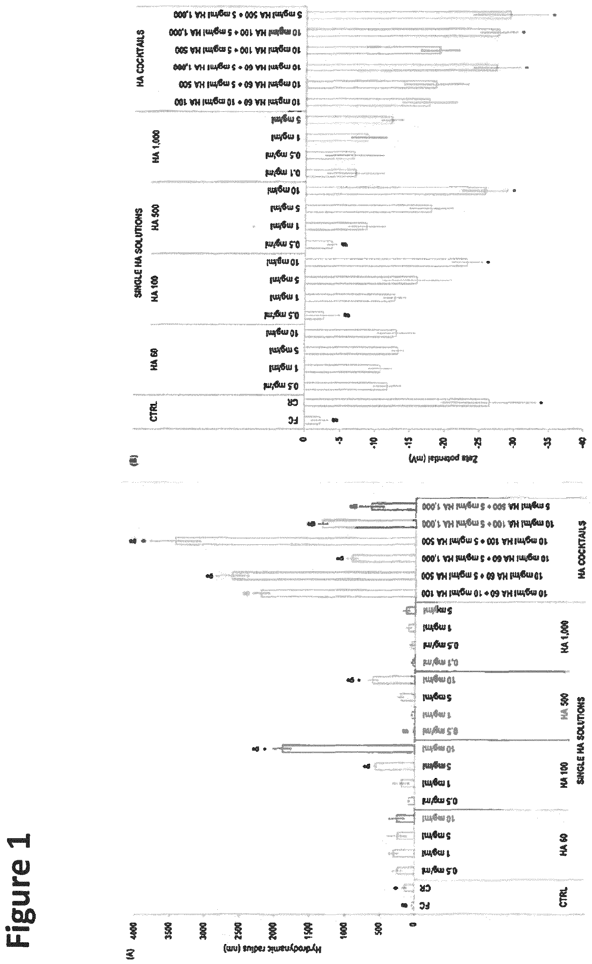

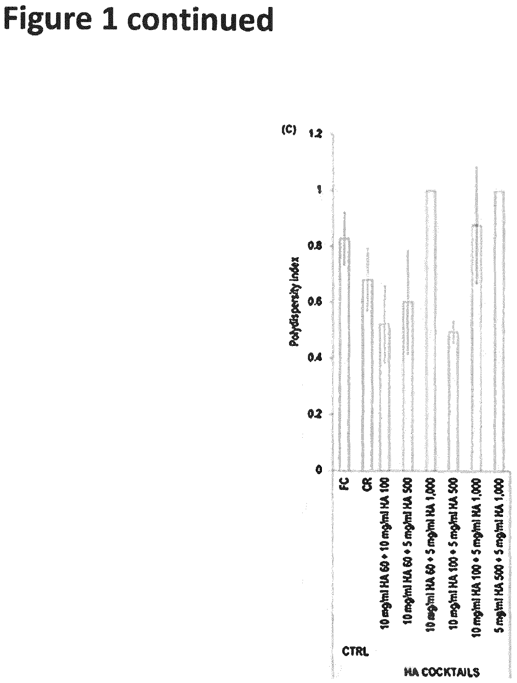

With respect to the hydrodynamic radius of single HA molecules (**Figure 1A**), the 5 and 10 mg/ml concentrations of the HA 100 kDa and the 10 mg/ml of the HA 500 kDa had significantly (p < 0.05) higher hydrodynamic radius than the CR and FC. The 10 mg/ml HA 100 kDa exhibited the highest (p < 0.05) hydrodynamic radius among all HA single concentrations, CR and FC. When the hydrodynamic radius of HA cocktails was assessed, all HA cocktails had significantly (p < 0.05) higher hydrodynamic radius than the CR and FC and the highest (p < 0.05) hydrodynamic radius was obtained for the 10 mg/ml HA 100 kDa + 5 mg/ml HA 500 kDa cocktail.

With respect to the zeta potential of single HA molecules (**Figure 1B**), all molecules had negative charge and the lowest (p < 0.05) charge was detected for CR and the 10 mg/ml of the 100 and 500 kDa HA. When the zeta potential of HA cocktails was assessed, the 10 mg/ml HA 60 kDa + 5 mg/ml HA 1,000 kDa, 10 mg/ml HA 100 kDa + 5 mg/ml HA 1,000 kDa and 5 mg/ml HA 500 kDa + 5 mg/ml HA 1,000 kDa cocktails induced significantly (p < 0.05) lower charge than the other groups.

With respect to the polydispersity index of the single HA molecules (**Figure 1C**), the 1 mg/ml and 5 mg/ml HA 100 kDa and all concentrations of the HA 60 kDa, 500 kDa and 1,000 kDa had polydispersity index higher than 0.6 (as did the FC and CR as well).When the polydispersity index of HA cocktails was assessed, only the 10 mg/ml HA 60 kDa + 5 mg/ml HA 1,000 kDa, 10 mg/ml HA 100 kDa + 5 mg/ml HA 1,000 kDa and 5 mg/ml HA 500 kDa + 5 mg/ml HA 1,000 kDa cocktails had polydispersity index higher than 0.6.

### 3.3. Cell morphology and ECM deposition analyses of single HA molecules

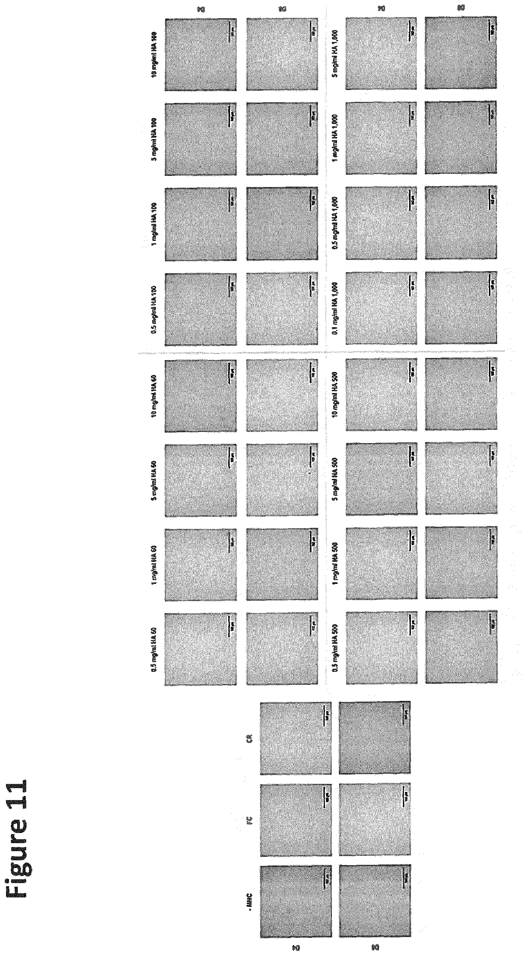

Qualitative bright field microscopy analysis did not reveal any differences in cell morphology as a function of MMC, independently of which molecule and at which concentration was used **(****Figure 11****).**

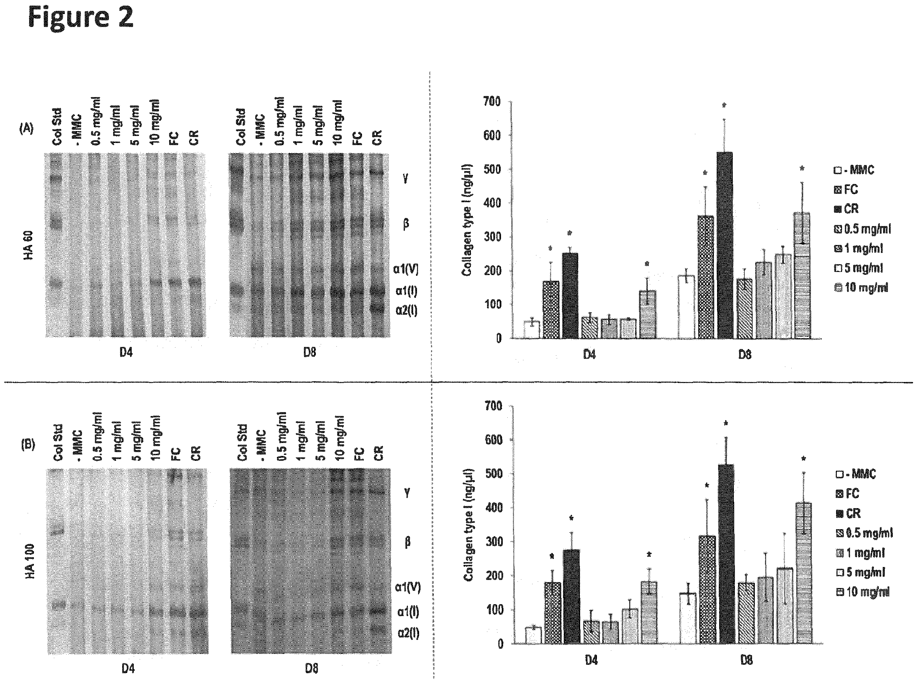

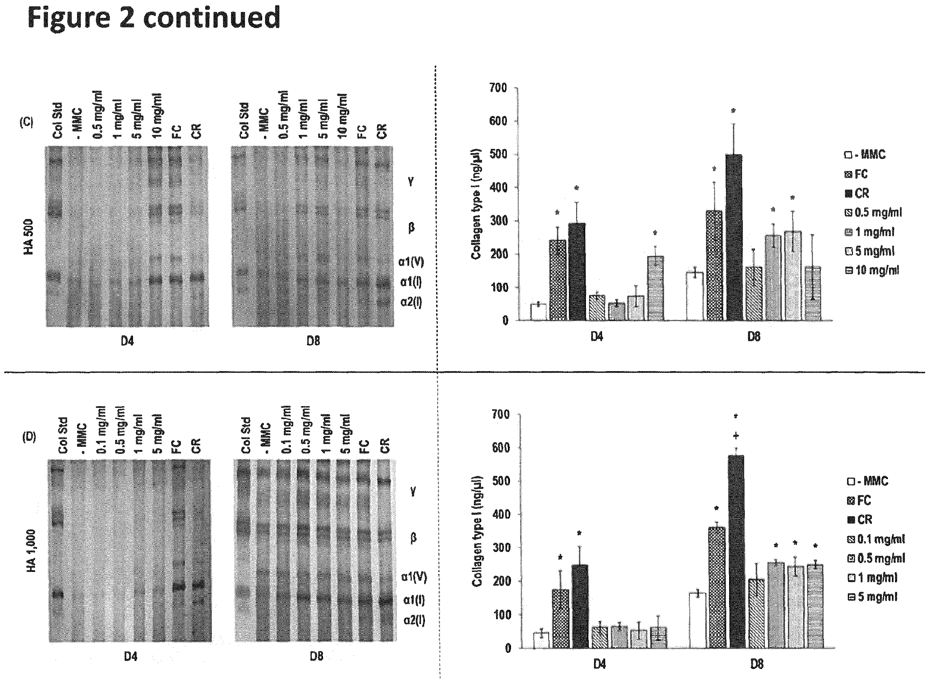

SDS-PAGE and densitometry analyses of the single HA molecules revealed that at both time points, the FC, CR and 10 mg/ml HA 60 kDa induced significantly (p < 0.05) higher collagen type I deposition than the non MMC group **(****Figure 2A****);** at both time points, the FC, CR and 10 mg/ml HA 100 kDa induced significantly (p < 0.05) higher collagen type I deposition than the non MMC group **(****Figure 2B****);** at both time points, the FC and CR and at day 4, the 10 mg/ml and at day 8, the 1 and 5 mg/ml HA 500 kDa induced significantly (p < 0.05) higher collagen type I deposition than the non MMC group **(****Figure 2C****);** and at both time points, the FC and CR and at day 8, the 0.5, 1 and 5 mg/ml HA 1,000 kDa induced significantly (p < 0.05) higher collagen type I deposition than the non MMC group **(****Figure 2D****).**

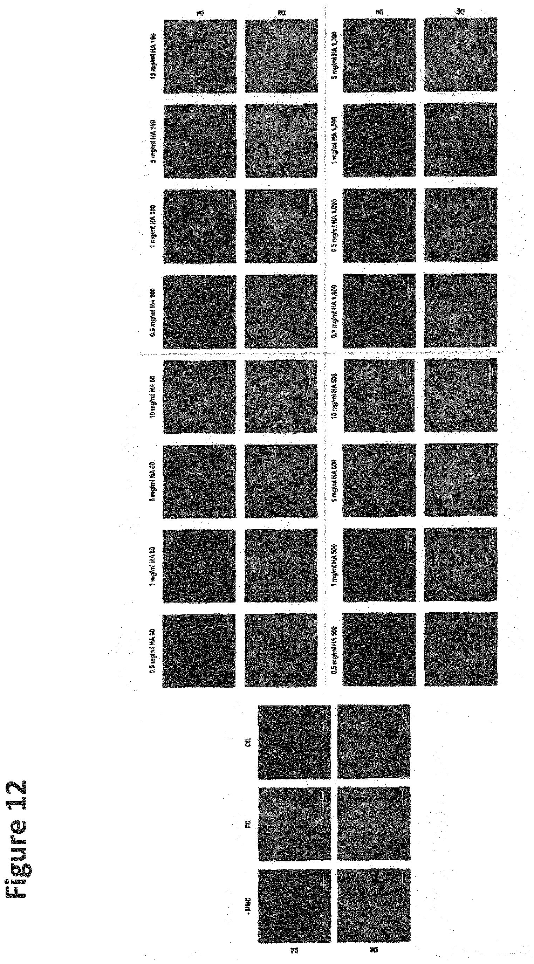

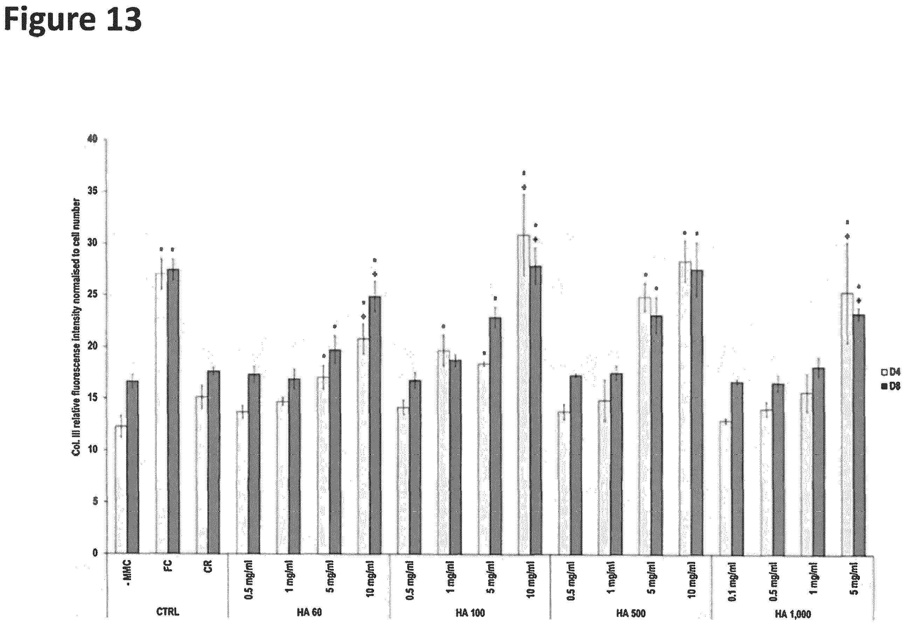

Immunocytochemistry **(****Figure 12****)** and image intensity **(****Figure 13****)** analyses of collagen type III made apparent that at both times, the FC; at both times, the 5 and 10 mg/ml HA 60 kDa; at day 4, the 1 mg/ml and at both time points, the 5 and 10 mg/ml HA 100 kDa; at both time points, the 5 and 10 mg/ml HA 500 kDa; and at both time points, the 5 mg/ml HA 1,000 induced significantly (p < 0.05) higher collagen type III deposition than the non MMC group.

Considering that the 10 mg/ml HA 60 kDa (significantly, p < 0.05, increased collagen type I and collagen type III at both time points, in comparison to the non MMC group), 10 mg/ml HA 100 kDa (significantly, p < 0.05, increased collagen type I and collagen type III at both time points, in comparison to the non MMC group), 5 mg/ml HA 500 kDa (significantly, p < 0.05, increased collagen type I at day 8 and collagen type III at both time points, in comparison to the non MMC group) and 5 mg/ml HA 1,000 kDa (significantly, p < 0.05, increased collagen type I at day 8 and collagen type III at both time points, in comparison to the non MMC group), these molecules and corresponding concentrations were selected to formulate HA cocktails.

### 3.4. Cell morphology analysis of HA cocktails

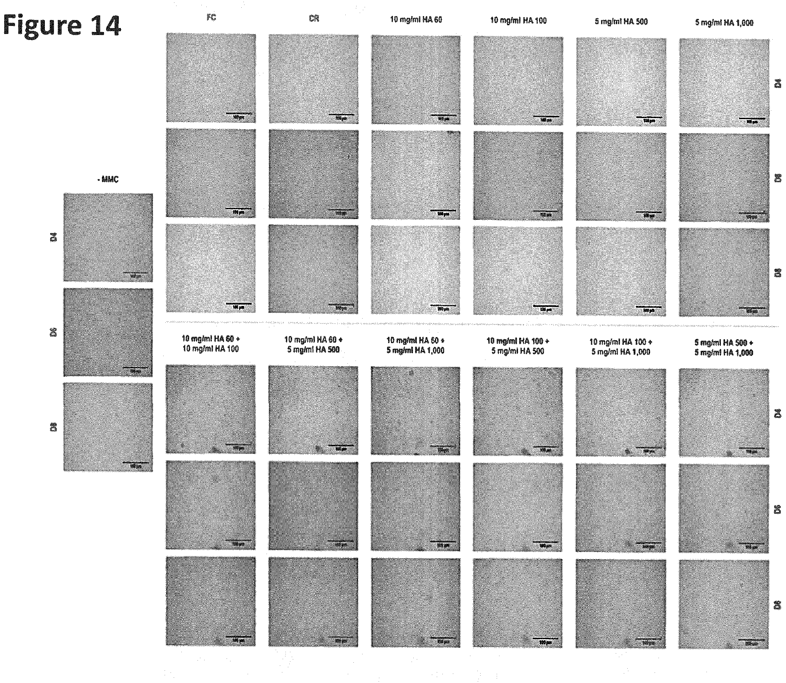

Qualitative bright field microscopy analysis did not reveal any differences in cell morphology as a function of MMC and time in culture, independently of which molecule (molecules) and at which concentration (s) was (were) used **(****Figure 14****).**

### 3.5. Cell viability, proliferation and metabolic activity analyses of HA cocktails

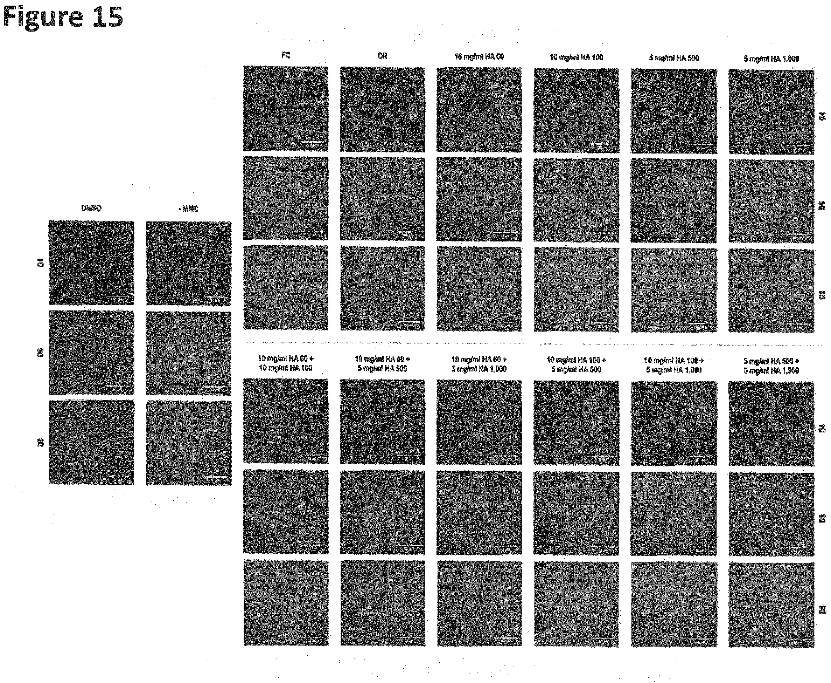

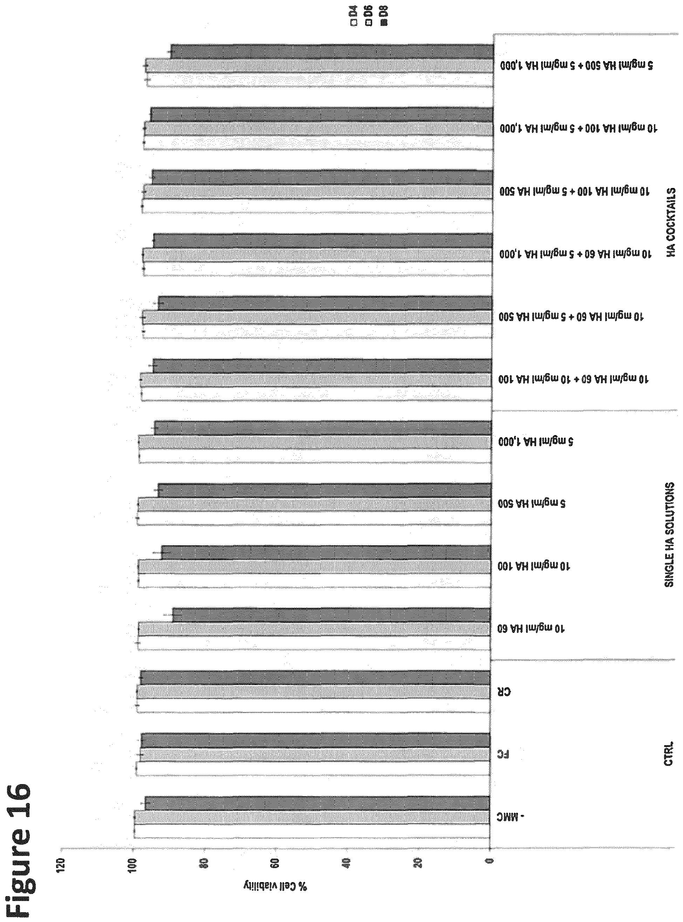

Qualitative **(****Figure 15****)** and quantitative **(****Figure 16****)** cell viability analyses revealed no apparent differences between the groups at any time point.

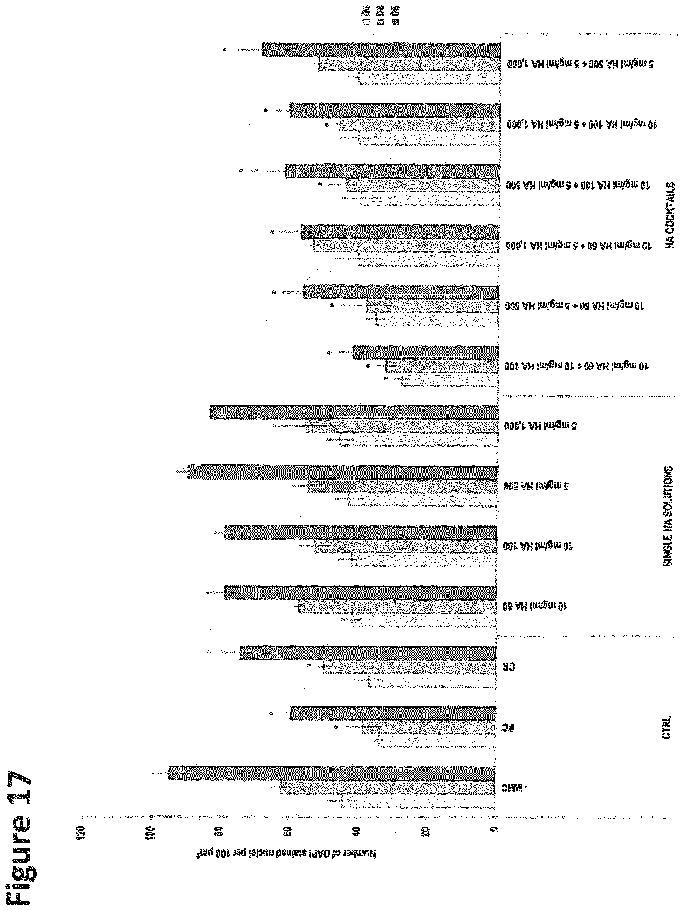

In comparison to the non MMC group at a given time point, a significant (p < 0.05) reduction in cell proliferation was observed with the FC at day 6 and day 8; CR at day 6; 10 mg/ml HA 60 kDa + 10 mg/ml HA 100 kDa cocktail at all time points; 10 mg/ml HA 60 kDa + 5 mg/ml HA 500 kDa cocktail at day 6 and day 8; 10 mg/ml HA 60 kDa + 5 mg/ml HA 1,000 kDa cocktail at day 8; 10 mg/ml HA 100 kDa + 5 mg/ml HA 500 kDa cocktail at day 6 and day 8; 10 mg/ml HA 100 kDa + 5 mg/ml HA 1,000 kDa cocktail at day 6 and day 8; and 5 mg/ml HA 500 kDa + 5 mg/ml HA 1,000 kDa cocktail at day 8 **(****Figure 17****).**

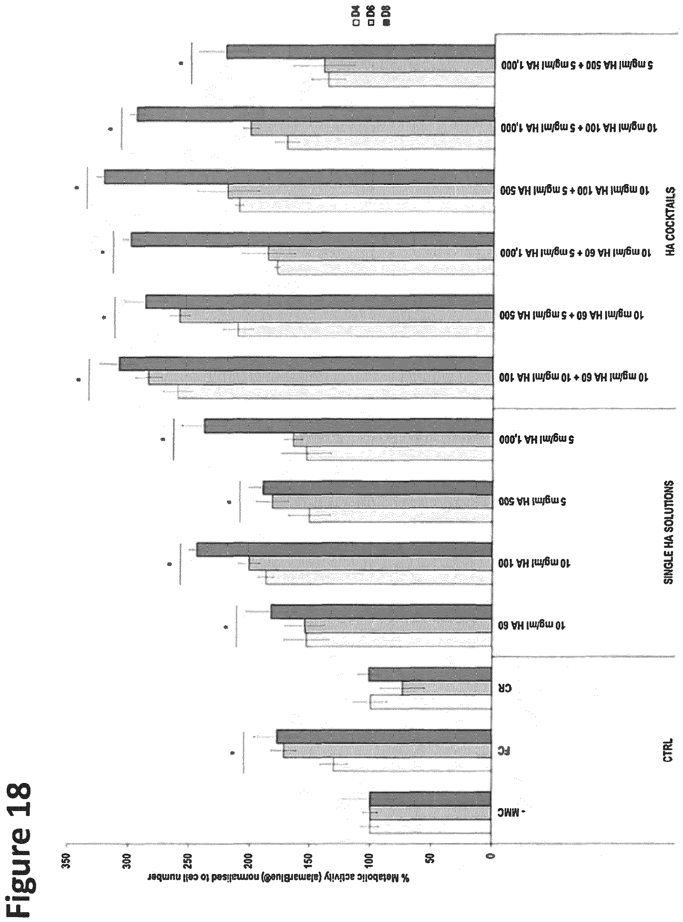

In comparison to the non MMC group at a given time point, a significant (p < 0.05) increase in cell metabolic activity normalised to cell proliferation was observed for all groups, apart from the CR group, which induced similar (p > 0.05) metabolic activity to the non MMC group **(****Figure 18****).**

### 3.6. ECM deposition analysis of HA cocktails

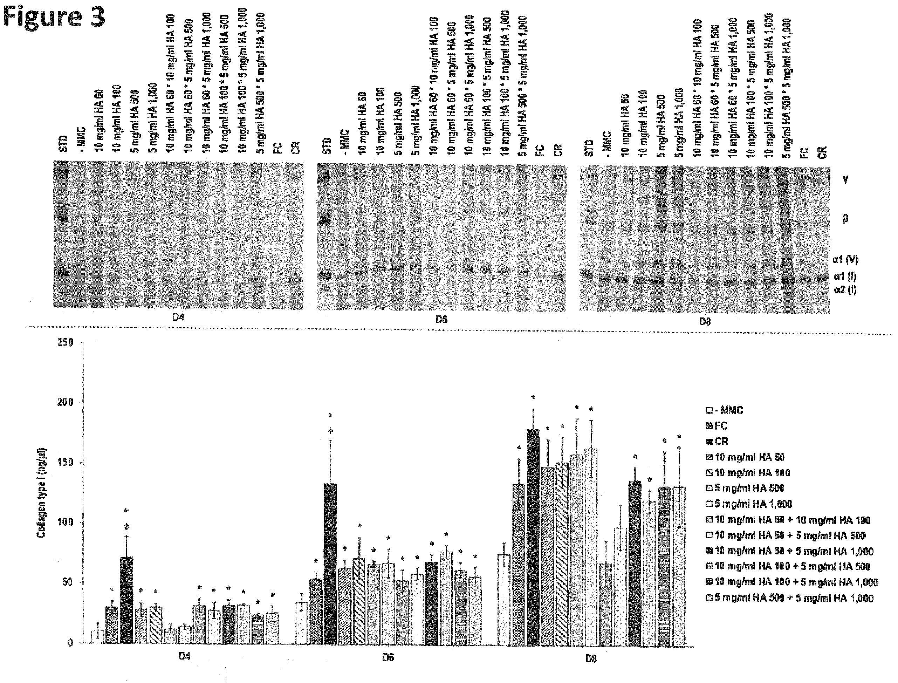

SDS-PAGE and complementary densitometry analyses **(****Figure 3****)** revealed that at day 4, all crowders, but the 5 mg/ml HA 500 and 1,000 kDa, significantly (p < 0.05) increased collagen type I deposition and CR induced the highest (p < 0.05) collagen type I deposition; at day 6, all crowders significantly (p < 0.05) increased collagen type I deposition and CR induced the highest (p < 0.05) collagen type I deposition; and at day 8, all crowders, but the 10 mg/ml HA 60 kDa + 10 mg/ml HA 100 kDa and the 10 mg/ml HA 60 kDa + 5 mg/ml HA 500 kDa, significantly (p < 0.05) increased collagen type I deposition.

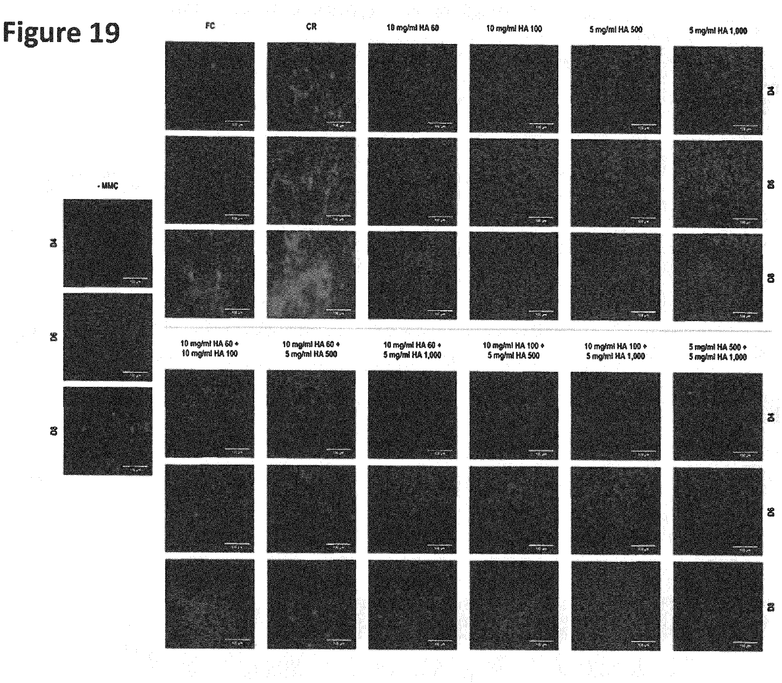

Immunocytochemistry **(****Figure 19****)** and complementary fluorescence intensity **(****Figure 4A****)** analyses for collagen type I revealed that only FC at day 8 and CR at all time points significantly (p < 0.05) increased collagen type I deposition, in comparison to non MMC group. CR induced the highest collagen type I deposition among all crowding conditions at all time points (p<0.05).

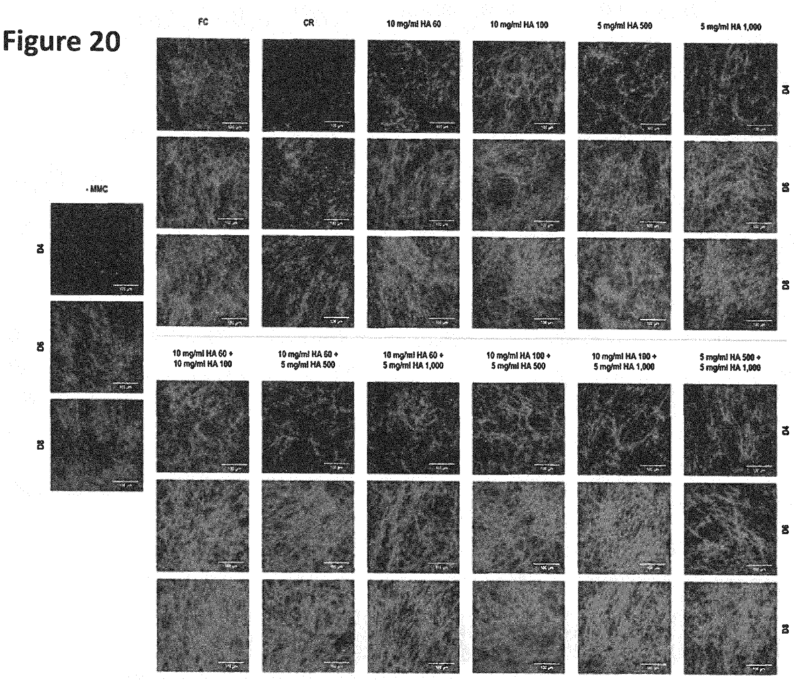

Immunocytochemistry **(****Figure 20****)** and complementary fluorescence intensity **(****Figure 4B****)** analyses for collagen type III revealed that at all time points, all crowders, but the CR, which induced similar (p > 0.05) collagen type III deposition to the non MMC group, significantly (p < 0.05) increased collagen type III deposition in comparison to non MMC group. In comparison to single HA solutions, at day 4, none of the HA cocktails induced significantly (p > 0.05) different collagen type III deposition; at day 6, all HA cocktails, but the 10 mg/ml HA 60 kDa + 5 mg/ml HA 1,000 kDa and the 5 mg/ml HA 500 kDa + 5 mg/ml HA 1,000 kDa, significantly (p < 0.05) increased collagen type III deposition; and at day 8, all HA cocktails, but the 10 mg/ml HA 100 kDa + 5 mg/ml HA 500 kDa and the 5 mg/ml HA 500 kDa + 5 mg/ml HA 1,000 kDa, significantly (p < 0.05) increased collagen type 111 deposition.

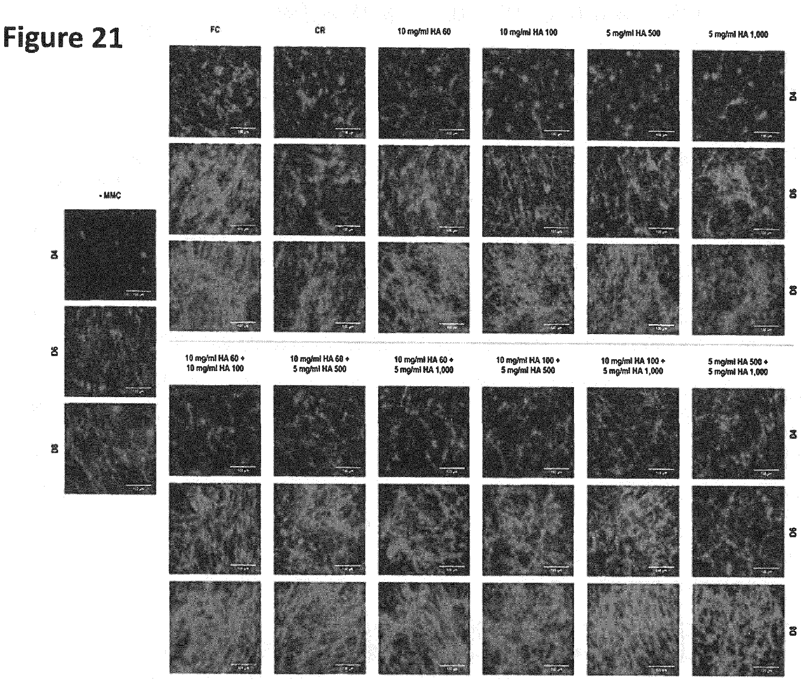

Immunocytochemistry **(****Figure 21****)** and complementary fluorescence intensity **(****Figure 4C****)** analyses for collagen type IV revealed that at all time points, all crowders significantly (p < 0.05) increased collagen type IV deposition in comparison to the non MMC group. In comparison to single HA solutions, at day 4 and day 6, none of the HA cocktails induced significantly (p > 0.05) different collagen type IV deposition and at day 8, the 10 mg/ml HA 60 kDa + 10 mg/ml HA 100 kDa, the 10 mg/ml HA 60 kDa + 5 mg/ml HA 500 kDa and 10 mg/ml HA 100 kDa + 5 mg/ml HA 500 kDa induced significantly (p < 0.05) higher collagen type IV deposition.

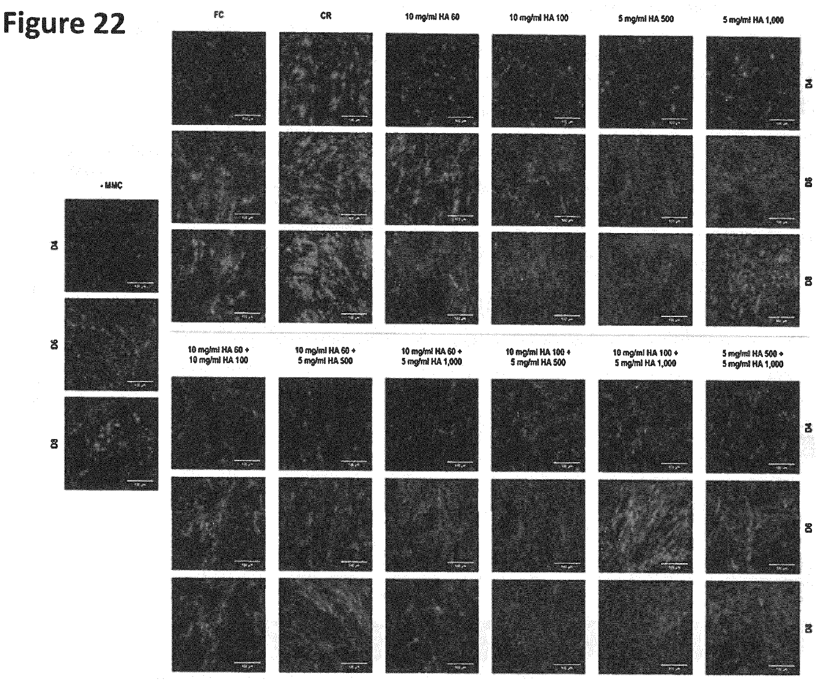

Immunocytochemistry **(****Figure 22****)** and complementary fluorescence intensity **(****Figure 4D****)** analyses for collagen type V revealed that FC at day 6 and 8; CR at all time points; the 10 mg/ml HA 60 kDa at day 6; the 10 mg/ml HA 60 kDa + 10 mg/ml HA 100 kDa cocktail at day 6; and the 10 mg/ml of the HA 100 kDa + 5 mg/ml of the HA 1000 kDa cocktail at day 6 significantly (p < 0.05) increased collagen type V deposition in comparison to the non MMC group. CR induced the highest (p < 0.05) collagen type V deposition among all groups at a given time point.

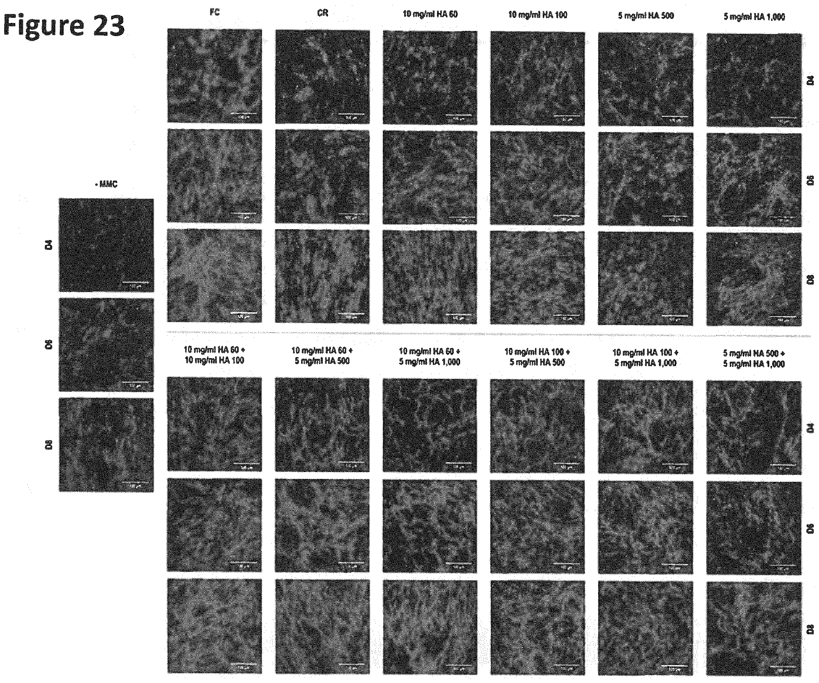

Immunocytochemistry **(****Figure 23****)** and complementary fluorescence intensity **(****Figure 4E****)** analyses for collagen type VI revealed that at day 4, all crowders, but the 5 mg/ml HA 1000 kDa and the 5 mg/ml HA 500 kDa + 5 mg/ml HA 1000 kDa cocktail, significantly (p < 0.05) increased collagen type VI deposition in comparison to the non MMC group; at day 6, all crowders, but the 10 mg/ml HA 60 kDa, the 10 mg/ml HA 100 kDa, the 5 mg/ml HA 500 kDa, the 5 mg/ml HA 1,000 kDa and the 5 mg/ml HA 500 kDa + 5 mg/ml HA 1,000 kDa cocktail, significantly (p < 0.05) increased collagen type VI deposition in comparison to the non MMC group; and at day 8, all crowders, but the 10 mg/ml HA 60 kDa, the 5 mg/ml HA 500 kDa and the 5 mg/ml HA 1,000 kDa, significantly (p < 0.05) increased collagen type VI deposition in comparison to the non MMC group. In comparison to single HA solutions, at day 8, the 10 mg/ml HA 60 kDa + 10 mg/ml HA 100 kDa cocktail significantly (p < 0.05) increased collagen type VI deposition.

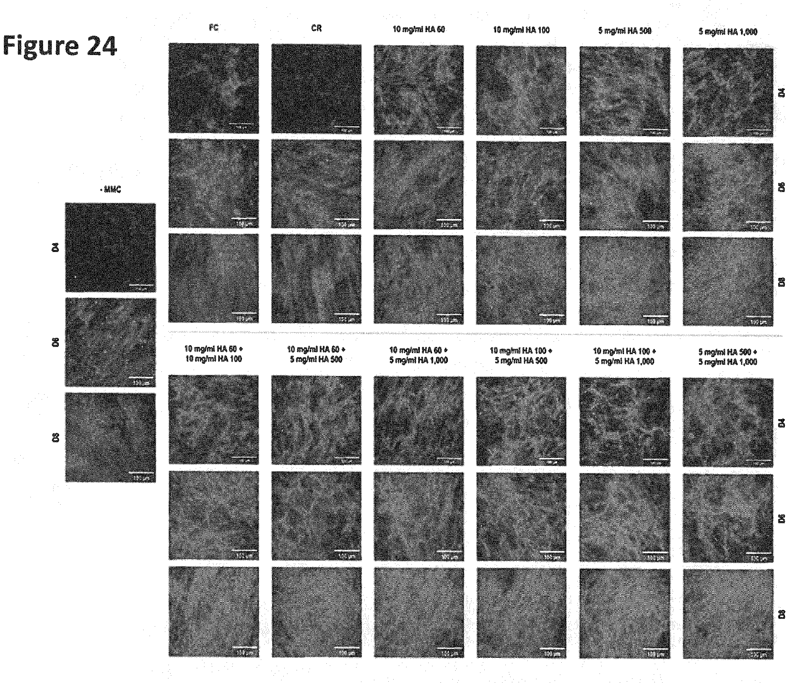

Immunocytochemistry **(****Figure 24****)** and complementary fluorescence intensity **(****Figure 4F****)** analyses for laminin revealed that at day 4, all crowders, but the CR, significantly (p < 0.05) increased laminin deposition in comparison to the non MMC group; at day 6, the 5 mg/ml HA 1,000 kDa and all HA cocktails significantly (p < 0.05) increased laminin deposition in comparison to the non MMC group; and at day 8, only the HA cocktails significantly (p < 0.05) increased laminin deposition in comparison to the non MMC group.

### 3.7. Tri-linage analysis of HA cocktails

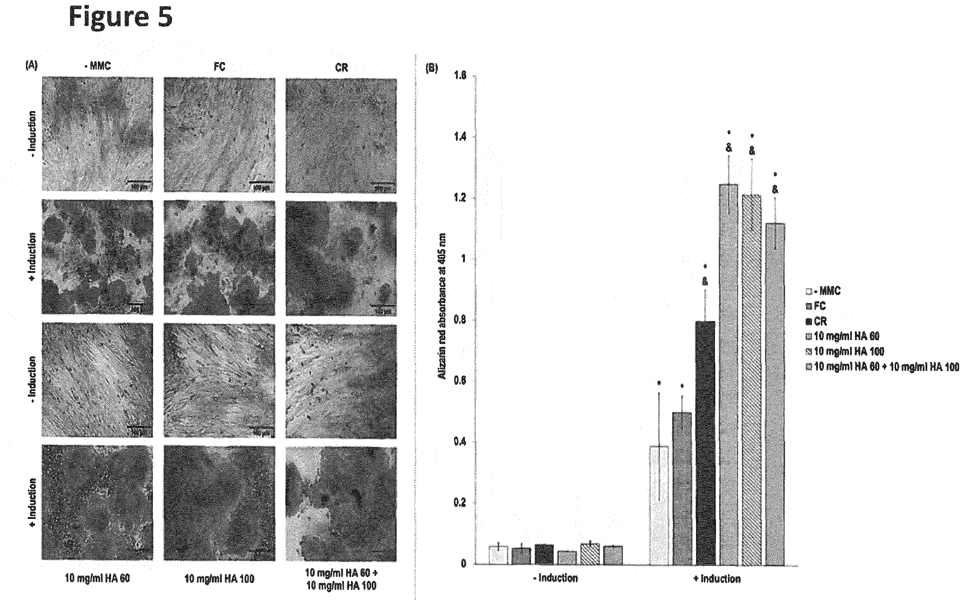

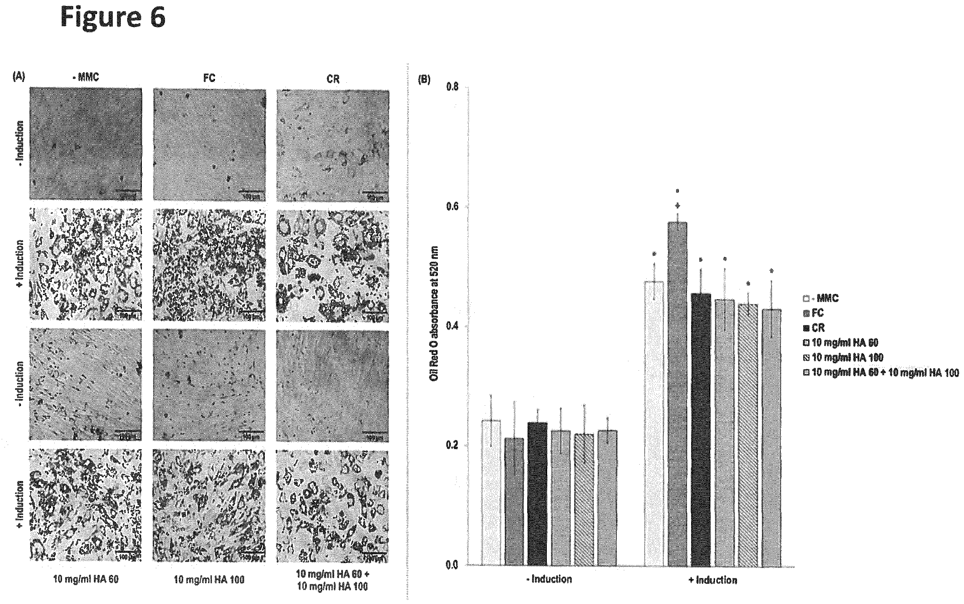

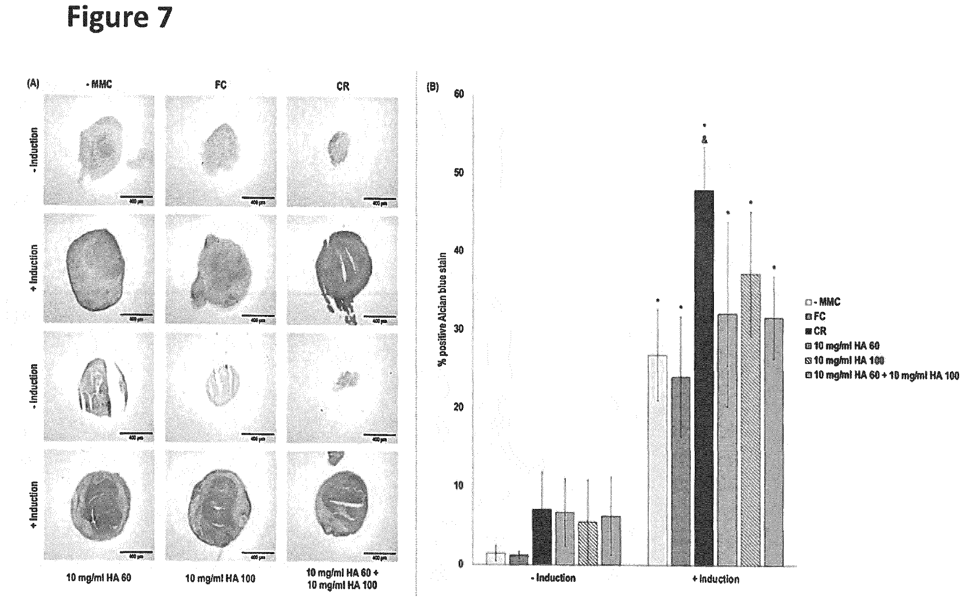

The 10 mg/ml HA 60 kDa + 10 mg/ml HA 100 kDa cocktail significantly (p < 0.05) increased collagen type III (at days 6 and 8), collagen type IV (at day 8) and collagen type VI (at day 8) over all single HA solutions. Thus, it was selected along with its individual components, FC and CR to assess tri-lineage differentiation. Osteogenic differentiation assessment **(****Figure 5A** for alizarin red staining and **Figure 5B** for calcium deposition) made apparent that the CR and the HA groups induced significantly (p < 0.05) higher mineralisation in comparison to the non MMC group and between them, the HA groups induced significantly (p < 0.05) higher mineralisation in comparison to the CR group, under osteogenic induction. Adipogenic differentiation assessment **(****Figure 6A**for red oil O staining and **Figure 6B** for red oil O staining quantification) made apparent that the FC induced the highest (p < 0.05) lipid droplets deposition under adipogenic induction. Chondrogenic differentiation assessment using Alcian blue staining **(****Figure 7A** for histology and **Figure 7B** for quantification) made apparent thatthe CR significantly (p < 0.05) increased the deposition of GAGs in comparison to the non MMC group under chondrogenic induction.

### 4. Discussion

MMC has been shown to dramatically enhance and accelerate ECM deposition in eukaryotic cell culture [55-57], resulting in far more functional tissue moduli for therapeutic purposes [58] and pathophysiology models for drug discovery purposes [59, 60]. Up to now, the vast majority of published work is dealing with single-macromolecule MMC systems and only a handful of studies have assessed the potential of mixed-macromolecule homo- [27] or hetero- [61] MMC systems. Herein, we ventured to assess the physicochemical properties and biological consequences in eBM-MSC cultures of single and mixed HA molecules and correlate them to the most widely used MMC agents, the FC cocktail and CR.

### 4.1. Single HA biophysical analysis and cell morphology and deposited ECM analyses

DLS analysis revealed a concentration-dependent increase of hydrodynamic radius for the HA 100 KDa, 500 kDa and 1,000 kDa, which is in agreement with a previous work [62]. This can be explained considering that in less concentrated solutions, soft interaction avoid macromolecule overlapping, whilst in more concentrated solutions, steric compression replaces the initial steric repulsion and the macromolecules become entangled [61]. With respect to HA 60 kDa, the hydrodynamic radius was not affected as a function of increased concentration. For this, we believe that the size of the molecule was too small to induce any changes. This again is in agreement with a previous work, where substantial changes in hydrodynamic radius were observed above certain molecular weights of specific macromolecules and significantly lower concentrations were needed for high in molecular weight macromolecules to induce significantly higher hydrodynamic radius changes than low in molecular weight macromolecules [64]. Similarly to hydrodynamic radius, a concentration-dependent increase of negative charge (or decrease of zeta potential) for the HA 100 KDa, 500 kDa and 1,000 kDa, which aligns with a previous work [65] and can be explained considering that zeta potential decreases when the ionic strength increases. With respect to HA 60 kDa, the zeta potential was not affected with concertation; although it was not specifically assessed herein, it has been argued that the zeta potential does not depend on the particle concentration, when the ratio of the number of bulk ions to the particle number is not lower than a certain value, subject to the particle diameter [66]. With only exception the 0.5 mg/ml and 10 mg/ml HA 100 kDa, all other concentrations of the different molecular weights HAs had polydispersity index higher than 0.6, which in general indicates samples with very broad particle size distribution (i.e. polydispersed populations), whilst samples with polydispersity index lower than 0.05 are considered as homogeneous (i.e. monodispersed populations) [67]. With respect to CR and FC, they exhibited hydrodynamic radius, zeta potential and polydispersity index values similar to those that have been reported previously in the literature [27, 32, 36]. In comparison to the different concentrations of the different molecular weight HAs, both FC and CR had significantly lower hydrodynamic radius than all concentrations of HA 60 kDa and the 5 mg/ml and the 10 mg/ml concentrations of HA 100 kDa and HA 500 kDa, which can be attributed to molecular weight and/or concentration differences [68-71]. With regards to zeta potential, in general, the FC is considered as neutral [72, 73] and had the lowest negative charge, whilst the CR, being sulphated [74], had the highest negative charge. With respect to polydispersity index, in general, both FC and CR had similar high polydispersity to HAs; the CR is naturally polydispersed [26], whilst the FC, being a cocktail of 37.5 mg/ml FC 70 KDa and 25 mg/ml FC 400 KDa, also exhibited high polydispersity, as has been shown before [26], in accordance with the mixed crowding theory [18, 19, 75]. At this point, we also feel important to point out that hydrodynamic radius and polydispersity index data should be cautiously assessed and contrasted at a given molecule's optimal concentration, as high concentrations may result in multi-scattering or agglomeration and low concentrations may not generate enough light to analyse [76, 17].

With respect to biological analyses and starting with qualitative cell morphology analysis, no differences were observed between the groups, which is in accordance with previous publications, where CR [27, 29] or FC [27, 78] or HA [35, 36] supplementation did not affect cell morphology. Based on SDS-PAGE for collagen type I deposition and immunocytochemistry for collagen type III deposition data, the 10 mg/ml HA 60 kDa, 10 mg/ml HA 100 kDa, 5 mg/ml HA 500 kDa and 5 mg/ml HA 1,000 kDa were selected to formulate HA cocktails, as they induced the highest (among the HAs) ECM deposition, and to be compared to FC and CR, the most widely used MMC agents. Considering that previous studies have shown high negative charge and polydispersity to be key modulators of ECM deposition [27], the selected concentrations of the different in molecular weight HAs fit well this theory. Further, the selected concentrations fit well the MMC agent concentration-dependent excluded volume effect theory [79]; as the concentration of a given MMC agent increases, the available volume decreases until the optimal concentration is reached, beyond which no further changes occur or the phenomenon is stopped altogether. For example, for CR, subject to the cell population used, the optimal concentration is between 50 and 100 µg/ml [26, 27, 30]. It is worth noting that CR outperformed, with respect to collagen type I deposition, all single HAs and the FC cocktail, whilst FC and the high concentrations of the HAs outperformed CR in collagen type III deposition. This selective collagen type deposition subject to the molecule used remains unexplained, albeit in agreement with previous observations [36], suggestive that the chemistry of the MMC agents should be considered in the selection of the crowding agents for a specific application and that the MMC agents are not inert, as frequently assumed [10, 28]. Finally, the data obtained herein with eBM-MSCs and CR, FC and the different concentrations of the different molecular weight HAs follow the same trends with data that we obtained previously with equine adipose derived MSCs [36], further demonstrating the reproducibility of the experimental approach.

### 4.2. HA cocktails biophysical analysis and cell morphology, basic function, deposited ECM and tri-lineage analyses

Starting with DLS analysis, the 1,000 kDa HA-based cocktails (with 60 kDa, 100 kDa or 500 kDa) induced the lowest hydrodynamic radius and the highest zeta potential and polydispersity index (in comparison to 60 kDa / 100 kDa, 60 kDa / 500 kDa, 100 kDa / 500 kDa cocktails). All HA cocktails, but the 100 kDa / 1,000 kDa cocktail, induced significantly higher hydrodynamic radius than the constituent HA molecules and the 1,000 kDa HA cocktails induced significantly higher zeta potential and polydispersity index than the constituent HA molecules. Lastly, in comparison to CR and FC, all HA cocktails induced significantly higher hydrodynamic radius and the 1,000 kDa, HA-based cocktails (with 60 kDa, 100 kDa or 500 kDa) matched the zeta potential of CR and outperformed both CR and FC with respect to polydispersity index. All these observations directly align with the excluding volume effect and mixed MMC agents theories, where biochemical reactions and biological processes have been shown to depend, among others, on the charge [16, 80], molecular length / size [81-83], concentration [14, 84] and shape [85-88] of the solutes.

Continuing with biological analyses, in general, qualitative cell morphology and quantitative basic cellular function analyses revealed that none of the MMC agents had any adverse effect on cell growth, which directly aligns with previous studies with CR [27, 29], FC [27, 78] and HA [35, 36]. With respect to ECM deposition, SDS-PAGE analysis showed that CR outperformed all others crowders at day 4 and day 6 and all crowders, but the cocktails of 10 mg/ml HA 60 kDa / 10 mg/ml HA 100 kDa and 10 mg/ml HA 60 kDa / 5 mg/ml HA 500 kDa, equally increased collagen type 1 deposition at day 8. Immunocytochemistry analyses revealed for collagen type I and collagen type V that CR outperformed all other MMC molecules at all time points; for collagen type III that FC, the individual HA molecules and the HA cocktails outperformed CR and that the HA cocktails outperformed their constituent HA molecules at all time points; for collagen type IV that FC outperformed CR and the individual HA molecules at all time points and that the HA cocktails outperformed their constituent HA molecules at day 6 and day 8; and for laminin that all individual HA molecules outperformed FC, which outperformed CR at day 4 and that, in general, the HA cocktails outperformed their constituent HA molecules at all time points. Based on previous observations [27], one would have expected the highest in negative charge and polydispersity index HA cocktails (e.g. 10 mg/ml HA 60 kDa / 5 mg/ml HA 1,000 kDa or 5 mg/ml HA 500 kDa / 5 mg/ml HA 1,000 kDa) to have induced the highest ECM deposition, as they would have more effectively excluded volume and reduced diffusion. This was not the case; instead, the 10 mg/ml HA 60 kDa / 10 mg/ml HA 100 kDa, which had lower zeta potential and polydispersity index, but higher hydrodynamic radius than the aforementioned HA cocktails significantly increased collagen type III, collagen type IV and collagen type VI deposition over them. Of course, none of them outperformed CR in collagen type I and collagen type V deposition, despite the fact CR having lower hydrodynamic radius, but similarly high polydispersity index and zeta potential. Collectively, all these observations suggest that all physicochemical characteristics (e.g. charge [16, 80], molecular length / size [81-83], concentration [14, 84], shape [85-88]) of the crowding agents should be accounted for, as well as the targeted enzymatically processed ECM protein. It should also be noted that, in general, all collagen types were either increased or remained constant as a function of time in culture, which has been observed previously and attributed to negative feedback loop phenomenon [89-9]]. Laminin, on the other hand, was decreased as a function of time in culture, which is also in agreement with previous studies with vascular or corneal endothelial cells, where laminin synthesis was significantly higher when the cells were sparse and actively growing than when the cells were confluent [92]. With regards to tri-lineage analyses, it is interesting to note that CR and HA (either as individual molecules or cocktail) significantly increased osteogenesis and chondrogenesis over FC and the FC significantly increased adipogenesis over CR and HA (either as individual molecules or cocktail). Once more, these observations are in accordance with previous publications, where sulphated polysaccharides, like CR, have been shown to enhance osteogenic and chondrogenic lineage commitment [31] via BMP-2 [93] or BMP-2-Smad-1/5/8 [94] and TGF-β1 [95, 96] or TGF-β3 [97], respectively, signalling. HA has been shown to also increase osteogenic and chondrogenic differentiation via the TGF-β/Smad [40] and Sox9 and ERK/Smad [98], respectively, signalling pathways. FC, on the other hand, has been shown to enhance adipogenic induction possibly via growth factor sequestration [33, 34].

### 5. Conclusions

Mixed macromolecular crowding has been proposed as a means to more effectively exclude volume. Herein we assessed the physicochemical properties and biological consequences in equine bone marrow mesenchymal stromal cell cultures of single and mixed HA molecules and correlated them to the Ficoll^{®} cocktail and carrageenan, the two most widely used macromolecular crowding agents. Hydrodynamic radius, zeta potential and polydispersity index were found to be molecule and concentration dependent. None of the assessed molecules affected basic cellular functions. With respect to extracellular matrix deposition, carrageenan outperformed all molecules with respect to collagen type ! and collagen type V deposition; the Ficoll^{®} cocktail and the hyaluronic acid cocktails outperformed carrageenan in collagen type III deposition; the hyaluronic acid cocktails outperformed the constituent molecules in collagen type IV and collagen type VI deposition; and the Ficoll^{®} cocktail and all hyaluronic acid molecules outperformed carrageenan in laminin deposition. Collectively, these data advocate the use of mixed crowding in eukaryotic cell culture and further support the notion that macromolecular crowders are not inert macromolecules.

The specific combinations of molecular weights and concentrations of HA used in accordance with the examples herein may be considered to be an embodiment of the composition of the invention either alone, or in combination with any cell culture medium.

### 6. References

[1] R.J. Ellis, A.P. Minton, Cell biology: Join the crowd, Nature 425(6953) (2003) 27-28.
[2] S. Zimmerman, S. Trach, Estimation of macromolecule concentrations and excluded volume effects for the cytoplasm of Escherichia coli, J Mol Biol 222(3) (1991) 599-620.
[3] G. Bit-Babik, A. Guy, C. Chou, A. Faraone, M. Kanda, A. Gessner, J. Wang, O. Fujiwara, Simulation of exposure and SAR estimation for adult and child heads exposed to radiofrequency energy from portable communication devices, Radiat Res 163(5) (2005) 580-590.
[4] D. Kapraun, J. Wambaugh, R. Setzer, R. Judson, Empirical models for anatomical and physiological changes in a human mother and fetus during pregnancy and gestation, PLoS One 14(5) (2019) e0215906.
[5] A.P. Minton, J. Wilf, Effect of macromolecular crowding upon the structure and function of an enzyme: Glyceraldehyde-3-phosphate dehydrogenase, Biochemistry 20(17) (1981) 4821-4826.
[6] G. Rivas, A. Minton, Macromolecular crowding in vitro, in vivo, and in between, Trends Biochem Sci 41(11) (2016) 970-981.
[7] N. Castaneda, C. Feuillie, M. Molinari, E. Kang, Actin bundle nanomechanics and organization are modulated by macromolecular crowding and electrostatic interactions, Front Mol Biosci 8 (2021) 760950.
[8] M. Sarkar, C. Li, G. Pielak, Soft interactions and crowding, Biophys Rev 5(2) (2013) 187-194.
[9] A.P. Minton, Models for excluded volume interaction between an unfolded protein and rigid macromolecular cosolutes: Macromolecular crowding and protein stability revisited, Biophys J 88(2) (2005) 971-985.
[10] A.P. Minton, Molecular crowding: Analysis of effects of high concentrations of inert cosolutes on biochemical equilibria and rates in terms of volume exclusion, Methods Enzymol 295 (1998) 127-149.
[11] M. Weiss, Crowding, diffusion, and biochemical reactions, in: R. Hancock, K.W. Jeon (Eds.), Int Rev Cel Mol Bio, Academic Press2014, pp. 383-417.
[12] M. Tabaka, T. Kalwarczyk, J. Szymanski, S. Hou, R. Holyst, The effect of macromolecular crowding on mobility of biomolecules, association kinetics, and gene expression in living cells, Front Phys 2(54) (2014) 00054.
[13] J.A. Dix, A.S. Verkman, Crowding effects on diffusion in solutions and cells, Annu Rev Biophys 37 (2008) 247-263.
[14] W. Jing, Y. Qin, J. Tong, Effects of macromolecular crowding on the folding and aggregation of glycosylated MUC5AC, Biochem Biophys Res Commun 529(4) (2020) 984-990.
[15] S. Biswas, P.K. Chowdhury, Unusual domain movement in a multidomain protein in the presence of macromolecular crowders, Phys Chem Chem Phys 17(30) (2015) 19820-19833.
[16] S. Palit, L. He, W. Hamilton, A. Yethiraj, A. Yethiraj, The effect of crowder charge in a model polymer-colloid system for macromolecular crowding: Polymer structure and dynamics, J Chem Phys 147(11) (2017) 1 14902.
[17] T. Kalwarczyk, K. Kwapiszewska, K. Szczepanski, K. Sozanski, J. Szymanski, B. Michalska, P. Patalas-Krawczyk, J. Duszynski, R. Holyst, Apparent anomalous diffusion in the cytoplasm of human cells: The effect of probes' polydispersity, J Phys Chem B 121(42) (2017) 9831-9837.
[18] B.R. Zhou, Y. Liang, F. Du, Z. Zhou, J. Chen, Mixed macromolecular crowding accelerates the oxidative refolding of reduced, denatured lysozyme: Implications for protein folding in intracellular environments, J Biol Chem 279(53) (2004) 55109-55116.
[19] S. Biswas, J. Kundu, S. Mukherjee, P. Chowdhury, Mixed macromolecular crowding: A protein and solvent perspective, ACS Omega 3(4) (2018) 4316-4330.
[20] Z. Lin, S. Li, Effect of mixed crowding on refolding of human muscle creatine kinase, Protein Pept Lett 17(11) (2010) 1426-1435.
[21] M. Gupta, P. Chowdhury, Protein dynamics as a sensor for macromolecular crowding: Insights into mixed crowding, J Mol Liq 347 (2022) 117969.
[22] D. Schachtschabel, J. Wever, Changes of glycosaminoglycan synthesis during in vitro ageing of human fibroblasts (WI-38), Aktuelle Gerontol 8(8) (1978) 403-409.
[23] R. Lareu, 1. Arsianti, H. Subramhanya, P. Yanxian, M. Raghunath, In vitro enhancement of collagen matrix formation and crosslinking for applications in tissue engineering: A preliminary study, Tissue Eng 13(2) (2007) 385-391.
[24] R. Lareu, K. Subramhanya, Y. Peng, P. Benny, C. Chen, Z. Wang, R. Rajagopalan, M. Raghunath, Collagen matrix deposition is dramatically enhanced in vitro when crowded with charged macromolecules: The biological relevance of the excluded volume effect, FEBS Lett 581(14) (2007) 2709-2714.
[25] C.Z. Chen, Y.X. Peng, Z.B. Wang, P.V. Fish, J.L. Kaar, R.R. Koepsel, A.J. Russell, R.R. Lareu, M. Raghunath, The Scar-in-a-Jar: Studying potential antifibrotic compounds from the epigenetic to extracellular level in a single well, British journal of pharmacology 158(5) (2009) 1196-1209.
[26] A. Satyam, P. Kumar, X. Fan, A. Gorelov, Y. Rochev, L. Joshi, H. Peinado, D. Lyden, B. Thomas, B. Rodriguez, M. Raghunath, A. Pandit, D. Zeugolis, Macromolecular crowding meets tissue engineering by self-assembly: A paradigm shift in regenerative medicine, Adv Mater 26(19) (2014) 3024-3034.
[27] D. Gaspar, K.P. Fuller, D.I. Zeugolis, Polydispersity and negative charge are key modulators of extracellular matrix deposition under macromolecular crowding conditions, Acta Biomater 88 (2019) 197-210.
[28] D. Hall, C. Dobson, Expanding to fill the gap: A possible role for inert biopolymers in regulating the extent of the 'macromolecular crowding' effect, FEBS Lett 580(1) (2006) 2584-2590.
[29] P. Kumar, A. Satyam, X. Fan, Y. Rochev, B.J. Rodriguez, A. Gorelov, L. Joshi, M. Raghunath, A. Pandit, D.I. Zeugolis, Accelerated development of supramolecular corneal stromal-like assemblies from corneal fibroblasts in the presence of macromolecular crowders, Tissue engineering. Part C, Methods 21(7) (2015) 660-670.
[30] D. Cigognini, D. Gaspar, P. Kumar, A. Satyam, S. Alagesan, C. Sanz-Nogues, M. Griffin, T. O'Brien, A. Pandit, D.I. Zeugolis, Macromolecular crowding meets oxygen tension in human mesenchymal stem cell culture - A step closer to physiologically relevant in vitro organogenesis, Sci Rep 6 (2016) 30746.
[31] V. Graceffa, D.I. Zeugolis, Carrageenan enhances chondrogenesis and osteogenesis in human bone marrow stem cell culture, Eur Cell Mater 37 (2019) 310-332.
[32] A. De Pieri, S. Rana, S. Korntner, D.I. Zeugolis, Seaweed polysaccharides as macromolecular crowding agents, Int J Biol Macromol 164 (2020) 434-446.
[33] X.M. Ang, M.H. Lee, A. Blocki, C. Chen, L.L. Ong, H.H. Asada, A. Sheppard, M. Raghunath, Macromolecular crowding amplifies adipogenesis of human bone marrow-derived mesenchymal stem cells by enhancing the pro-adipogenic microenvironment, Tissue engineering. Part A 20(5-6) (2014) 966-981.
[34] M.H. Lee, A.G. Goralczyk, R. Kriszt, X.M. Ang, C. Badowski, Y. Li, S.A. Summers, S.A. Toh, M.S. Yassin, A. Shabbir, A. Sheppard, M. Raghunath, ECM microenvironment unlocks brown adipogenic potential of adult human bone marrow-derived MSCs, Sci Rep 6 (2016) 21 173.
[35] D. Shendi, J. Marzi, W. Linthicum, A.J. Rickards, D.M. Dolivo, S. Keller, M.A. Kauss, Q. Wen, T.C. McDevitt, T. Dominko, K. Schenke-Layland, M.W. Rolle, Hyaluronic acid as a macromolecular crowding agent for production of cell-derived matrices, Acta Biomater 100 (2019) 292-305.
[36] S. Garnica-Galvez, S.H. Korntner, I. Skoufos, A. Tzora, N. Diakakis, N. Prassinos, D.I. Zeugolis, Hyaluronic acid as macromolecular crowder in equine adipose-derived stem cell cultures, Cells 10(4) (2021) 859.
[37] A. Hegewald, J. Ringe, J. Bartel, I. Krüger, M. Notter, D. Barnewitz, C. Kaps, M. Sittinger, Hyaluronic acid and autologous synovial fluid induce chondrogenic differentiation of equine mesenchymal stem cells: A preliminary study, Tissue Cell 36(6) (2004) 431-438.
[38] T. Tanaka, T. Furumatsu, S. Miyazawa, M. Fujii, H. Inoue, Y. Kodama, T. Ozaki, Hyaluronan stimulates chondrogenic gene expression in human meniscus cells, Connect Tissue Res 58(6) (2017) 520-530.
[39] G. Monaco, A. El Haj, M. Alini, M. Stoddart, Sodium hyaluronate supplemented culture media as a new hMSC chondrogenic differentiation media-model for in vitro/ex vivo screening of potential cartilage repair therapies, Front Bioeng Biotechnol 8 (2020) 243.
[40] L. Zhang, R. Liu, Y. Luo, Y. Zhao, D. Chen, C. Yu, J. Xiao, Hyaluronic acid promotes osteogenic differentiation of human amniotic mesenchymal stem cells via the TGF-β/Smad signalling pathway, Life Sci 232 (2019) 116669.
[41] F. Paolella, E. Gabusi, C. Manferdini, A. Schiavinato, G. Lisignoli, Specific concentration of hyaluronan amide derivative induces osteogenic mineralization of human mesenchymal stromal cells: Evidence of RUNX2 and COL1A1 genes modulation, J Biomed Mater Res A 107(12) (2019) 2774-2783.
[42] M. Asparuhova, V. Chappuis, A. Stähli, D. Buser, A. Sculean, Role of hyaluronan in regulating self-renewal and osteogenic differentiation of mesenchymal stromal cells and pre-osteoblasts, Clin Oral Investig 24(11) (2020) 3923-3937.
[43] J.M. McKim, J.A. Willoughby, W.R. Blakemore, M.L. Weiner, A critical review of "A randomized trial of the effects of the no-carrageenan diet on ulcerative colitis disease activity (Nutr. Healthy Aging. 2017; 4(2): 181-192).", J Nutr Health Aging 5 (2019) 149-158.
[44] Q. Shang, W. Sun, X. Shan, H. Jiang, C. Cai, J. Hao, G. Li, G. Yu, Carrageenan-induced colitis is associated with decreased population of anti-inflammatory bacterium, Akkermansia muciniphila, in the gut microbiota of C57BL/6J mice, Toxicol Lett 279 (2017) 87-95.
[45] Y. Mi, Y.X. Chin, W.X. Cao, Y.G. Chang, P.E. Lim, C.H. Xue, Q.J. Tang, Native κ-carrageenan induced-colitis is related to host intestinal microecology, Int J Biol Macromol 147 (2020) 284-294.
[46] M. Sharratt, P. Grasso, F. Carpanini, S. Gangolli, Carrageenan ulceration as a model for human ulcerative colitis, Lancet 2(7679) (1970) 932.
[47] M.L. Gutiérrez, J. Guevara, L.A. Barrera, Semi-automatic grading system in histologic and immunohistochemistry analysis to evaluate in vitro chondrogenesis, Universitas Scientiarum 17(2) (2012) 167-178.
[48] B. Ranera, A.R. Remacha, S. Alvarez-Arguedas, A. Romero, F.J. Vazquez, P. Zaragoza, I. Martin-Burriel, C. Rodellar, Effect of hypoxia on equine mesenchymal stem cells derived from bone marrow and adipose tissue, BMC Vet Res 8 (2012) 142.
[49] B. Ranera, J. Lyahyai, A. Romero, F.J. Vazquez, A.R. Remacha, M.L. Bernal, P. Zaragoza, C. Rodellar, 1. Martin-Burriel, Immunophenotype and gene expression profiles of cell surface markers of mesenchymal stem cells derived from equine bone marrow and adipose tissue, Veterinary immunology and immunopathology 144(1-2) (2011) 147-154.
[50] L. Maia, F.C. Landim-Alvarenga, L.S. Da Mota, M. De Assis Golim, R. Laufer-Amorim, B. De Vita, D.J. Barberini, A.J. Listoni, C.N. De Moraes, M.C. Heckler, R.M. Amorim, Immunophenotypic, immunocytochemistry, ultrastructural, and cytogenetic characterization of mesenchymal stem cells from equine bone marrow, Microscopy research and technique 76(6) (2013) 618-624.
[51] D.J. Barberini, N.P. Freitas, M.S. Magnoni, L. Maia, A.J. Listoni, M.C. Heckler, M.J. Sudano, M.A. Golim, F. da Cruz Landim-Alvarenga, R.M. Amorim, Equine mesenchymal stem cells from bone marrow, adipose tissue and umbilical cord: Immunophenotypic characterization and differentiation potential, Stem Cell Res Ther 5(1) (2014) 25.
[52] J. Fulber, D.A. Maria, L.C. da Silva, C.O. Massoco, F. Agreste, R.Y. Baccarin, Comparative study of equine mesenchymal stem cells from healthy and injured synovial tissues: An in vitro assessment, Stem Cell Res Ther 7 (2016) 35.
[53] M.K. Shikh Alsook, A. Gabriel, J. Piret, O. Waroux, C. Tonus, D. Connan, E. Baise, N. Antoine, Tissues from equine cadaver ligaments up to 72 hours of post-mortem: A promising reservoir of stem cells, Stem Cell Res Ther 6 (2015) 253.
[54] H. Capella-Monsonis, J.Q. Coentro, V. Graceffa, Z. Wu, D.I. Zeugolis, An experimental toolbox for characterization of mammalian collagen type I in biological specimens, Nat Protoc 13(3) (2018) 507-529.
[55] D. Tsiapalis, D. Zeugolis, It is time to crowd your cell culture media - Physicochemical considerations with biological consequences, Biomaterials 275 (2021) 120943.
[56] M. Raghunath, D. Zeugolis, Transforming eukaryotic cell culture with macromolecular crowding, Trends Biochem Sci 46(10) (2021) 805-811.
[57] D. Zeugolis, Bioinspired in vitro microenvironments to control cell fate: Focus on macromolecular crowding, Am J Physiol Cell Physiol 320(5) (2021) C842-C849.
[58] A. De Pieri, S. Korntner, H. Capella-Monsonis, D. Tsiapalis, S. Kostjuk, S. Churbanov, P. Timashev, A. Gorelov, Y. Rochev, D. Zeugolis, Macromolecular crowding transforms regenerative medicine by enabling the accelerated development of functional and truly three-dimensional cell assembled micro tissues, Biomaterials 287 (2022) 121674.
[59] J. Coentro, U. May, S. Prince, J. Zwaagstra, O. Ritvos, T. Järvinen, D. Zeugolis, Adapting the Scar-in-a-Jar to skin fibrosis and screening traditional and contemporary anti-fibrotic therapies, Front Bioeng Biotechnol 9 (2021) 756399.
[60] N. Shologu, M. Gurdal, E. Szegezdi, U. FitzGerald, D. Zeugolis, Macromolecular crowding in the development of a three-dimensional organotypic human breast cancer model, Biomaterials 287 (2022) 121642.
[61] M. Prewitz, A. Stißel, J. Friedrichs, N. Träber, S. Vogler, M. Bornhäuser, C. Werner, Extracellular matrix deposition of bone marrow stroma enhanced by macromolecular crowding, Biomaterials 73 (2015) 60-69.
[62] J. Yan-feng, C. Xu-long, Z. Yang-wen, X. Hui, S. Xiu-zhi, L. Hai-tao, Synthesis of super high molecular weight copolymer of AM/NaA/AMPS by oxidation-reduction and controlled radical polymerization, Pet Sci 17 (2020) 242-254.
[63] P. Blanco, J. Garcés, S. Madurga, F. Mas, Macromolecular diffusion in crowded media beyond the hard-sphere model, Soft Matter 14(16) (2018) 3105-3114.
[64] J. Armstrong, R. Wenby, H. Meiselman, T. Fisher, The hydrodynamic radii of macromolecules and their effect on red blood cell aggregation, Biophys J 87(6) (2004) 4259-4270.
[65] I. Ostolska, M. Wiśniewska, Application of the zeta potential measurements to explanation of colloidal Cr2O3 stability mechanism in the presence of the ionic polyamino acids, Colloid Polym Sci 292 (2014) 2453-2464.
[66] K. Medrzycka, The effect of particle concentration on zeta potential in extremely dilute solutions, Colloid Polym Sci 269 (1991) 85-90.
[67] M. Danaei, M. Dehghankhold, S. Ataei, F. Hasanzadeh Davarani, R. Javanmard, A. Dokhani, S. Khorasani, M. Mozafari, Impact of particle size and polydispersity index on the clinical applications of lipidic nanocarrier systems, Pharmaceutics 10(2) (2018) 57.
[68] N. Tokuriki, M. Kinjo, S. Negi, M. Hoshino, Y. Goto, I. Urabe, T. Yomo, Protein folding by the effects of macromolecular crowding, Protein Sci 13(1) (2004) 125-133.
[69] C. Le Coeur, J. Teixeira, P. Busch, S. Longeville, Compression of random coils due to macromolecular crowding: Scaling effects, Phys Rev E Stat Nonlin Soft Matter Phys 81(6 Pt 1) (2010) 061914.
[70] K. Sharp, Analysis of the size dependence of macromolecular crowding shows that smaller is better, Proc Natl Acad Sci U S A 112(26) (2015) 7990-7995.
[71] L. Stagg, S. Zhang, M. Cheung, P. Wittung-Stafshede, Molecular crowding enhances native structure and stability of alpha/beta protein flavodoxin, Proc Natl Acad Sci U S A 104(48) (2007) 18976-18981.
[72] A. Zeiger, F. Loe, R. Li, M. Raghunath, K. Van Vliet, Macromolecular crowding directs extracellular matrix organization and mesenchymal stem cell behavior, PLoS One 7(5) (2012) e37904.
[73] A. Marianelli, B. Miller, C. Keating, Impact of macromolecular crowding on RNA/spermine complex coacervation and oligonucleotide compartmentalization, Soft Matter 14(3) (2018) 368-378.
[74] L. Li, R. Ni, Y. Shao, S. Mao, Carrageenan and its applications in drug delivery, Carbohydr Polym 103 (2014) 1-11.
[75] H. Zhou, Effect of mixed macromolecular crowding agents on protein folding, Proteins 72(4) (2008) 1109-1113.
[76] S. Bhattacharjee, DLS and zeta potential - What they are and what they are not?, J Control Release 235 (2016) 337-351.
[77] J. Panchal, J. Kotarek, E. Marszal, E.M. Topp, Analyzing subvisible particles in protein drug products: A comparison of dynamic light scattering (DLS) and resonant mass measurement (RMM), AAPS J 16(3) (2014) 440-451.
[78] P. Kumar, A. Satyam, X. Fan, E. Collin, Y. Rochev, B. Rodriguez, A. Gorelov, S. Dillon, L. Joshi, M. Raghunath, A. Pandit, D. Zeugolis, Macromolecularly crowded in vitro microenvironments accelerate the production of extracellular matrix-rich supramolecular assemblies, Sci Rep 5 (2015) 8729.
[79] K. Suematsu, Concentration dependence of excluded volume effects, Colloid Polym Sci 290 (2012) 481-490.
[80] P. Blanco, S. Madurga, J. Garcés, F. Mas, R. Dias, Influence of macromolecular crowding on the charge regulation of intrinsically disordered proteins, Soft Matter 17(3) (2021) 655-669.
[81] D. Gomez, K. Huber, S. Klumpp, On protein folding in crowded conditions, J Phys Chem Lett 10(24) (2019) 7650-7656.
[82] N. Das, P. Sen, Size-dependent macromolecular crowding effect on the thermodynamics of protein unfolding revealed at the single molecular level, Int J Biol Macromol 141 (2019) 843-854.
[83] M. Vibhute, M. Schaap, R. Maas, F. Nelissen, E. Spruijt, H. Heus, M. Hansen, W. Huck, Transcription and translation in cytomimetic protocells perform most efficiently at distinct macromolecular crowding conditions, ACS Synth Biol 9(10) (2020) 2797-2807.
[84] P. Dey, A. Bhattacherjee, Role of macromolecular crowding on the intracellular diffusion of DNA binding proteins, Sci Rep 8(1) (2018) 844.
[85] N. Das, P. Sen, Macromolecular crowding: How shape and interaction affect the structure, function, conformational dynamics and relative domain movement of a multidomain protein, Phys Chem Chem Phys 24(23) (2022) 14242-14256.
[86] A. Guseman, G. Perez Goncalves, S. Speer, G. Young, G. Pielak, Protein shape modulates crowding effects, Proc Natl Acad Sci U S A 115(43) (2018) 10965-10970.
[87] T. Skóra, F. Vaghefikia, J. Fitter, S. Kondrat, Macromolecular crowding: How shape and interactions affect diffusion, J Phys Chem B 124(35) (2020) 7537-7543.
[88] N. Das, P. Sen, Shape-dependent macromolecular crowding on the thermodynamics and microsecond conformational dynamics of protein unfolding revealed at the single-molecule level, J Phys Chem B 124(28) (2020) 5858-5871.
[89] D. Tsiapalis, A. De Pieri, K. Spanoudes, I. Salient, S. Kearns, J. Kelly, M. Raghunath, D. Zeugolis, The synergistic effect of low oxygen tension and macromolecular crowding in the development of extracellular matrix-rich tendon equivalents, Biofabrication 12(2) (2020) 025018.
[90] C. Ryan, E. Pugliese, N. Shologu, D. Gaspar, P. Rooney, M. Islam, A. O'Riordan, M. Biggs, M. Griffin, D. Zeugolis, A combined physicochemical approach towards human tenocyte phenotype maintenance, Mater Today Bio 12 (2021) 100130.
[91] C. Ryan, E. Pugliese, N. Shologu, D. Gaspar, P. Rooney, M. Islam, A. O'Riordan, M. Biggs, M. Griffin, D. Zeugolis, The synergistic effect of physicochemical in vitro microenvironment modulators in human bone marrow stem cell cultures, Biomater Adv 144 (2023) 213196.
[92] D. Gospodarowicz, G. Greenburg, J. Foidart, N. Savion, The production and localization of laminin in cultured vascular and corneal endothelial cells, J Cell Physiol 107(2) (1981) 171-183.
[93] Y. Pan, J. Chen, Y. Yu, K. Dai, J. Wang, C. Liu, Enhancement of BMP-2-mediated angiogenesis and osteogenesis by 2-N,6-O-sulfated chitosan in bone regeneration, Biomater Sci 6(2) (2018) 431-439.
[94] B. Kim, H. Kang, J. Park, J. Lee, Fucoidan promotes osteoblast differentiation via JNK- and ERK-dependent BMP2-Smad 1/5/8 signaling in human mesenchymal stem cells, Exp Mol Med 47(1) (2015) e128.
[95] C. Merceron, S. Portron, C. Vignes-Colombeix, E. Rederstorff, M. Masson, J. Lesoeur, S. Sourice, C. Sinquin, S. Colliec-Jouault, P. Weiss, C. Vinatier, J. Guicheux, Pharmacological modulation of human mesenchymal stem cell chondrogenesis by a chemically oversulfated polysaccharide of marine origin: Potential application to cartilage regenerative medicine, Stem Cells 30(3) (2012) 471-480.
[96] N. Waghmare, A. Arora, A. Bhattacharjee, D. Katti, Sulfated polysaccharide mediated TGF-β1 presentation in pre-formed injectable scaffolds for cartilage tissue engineering, Carbohydr Polym 193 (2018) 62-72.
[97] J. Park, D. Woo, H. Yang, K. Na, K. Park, Transforming growth factor beta-3 bound with sulfate polysaccharide in synthetic extracellular matrix enhanced the biological activities for neocartilage formation in vivo, J Biomed Mater Res A 91(2) (2009) 408-415.
[98] Y. Luo, A. Wang, Q. Zhang, R. Liu, J. Xiao, RASL11B gene enhances hyaluronic acid-mediated chondrogenic differentiation in human amniotic mesenchymal stem cells via the activation of Sox9/ERK/smad signals, Exp Biol Med (Maywood) 245(18) (2020) 1708-1721.

All references are herein incorporated by reference.

## Claims

1. A composition for cell culture, the composition comprising glycosaminoglycan (GAG) having a molecular weight of from 60 to 2000 kDa, wherein the GAG has a molecular weight distribution comprising two or more peaks.

2. The composition according to claim 1, wherein the glycosaminoglycan is a negatively charged glycosaminoglycan.

3. The composition according to claim 1 or claim 2, wherein the glycosaminoglycans comprise or consist of hyaluronic acid (HA).

4. The composition according to any preceding claim, wherein the composition comprises:
GAG molecular weights in a range of from 66kDa to 99kDa and GAG molecular weights in the range of from 100kDa to 185kDa;
GAG molecular weights in a range of from 66kDa to 99kDa and GAG molecular weights in the range of from 301 to 465 kDa;
GAG molecular weights in a range of from 66kDa to 99kDa and GAG molecular weights in the range of from 1.01 to 1.80 MDa;
GAG molecular weights in a range of from 100kDa to 185kDa and GAG molecular weights in the range of from 301 to 465 kDa;
GAG molecular weights in a range of from 100kDa to 185kDa and GAG molecular weights in the range of from 1.01 to 1.80 MDa; or
GAG molecular weights in a range of from 301 to 465 kDa and GAG molecular weights in the range of from 1.01 to 1.80 MDa.

5. The composition according to any preceding claim, wherein first and second ranges of glycosaminoglycan are provided in a ratio of between 1:4 and 4:1 w/w.

6. The composition according to any preceding claim, wherein the composition is a cell culture composition comprising cell culture medium.

7. The composition according to claim 6, wherein the concentration of the glycosaminoglycans in the culture medium does not exceed 20 mg/ml.

8. The composition according to claim 6 or 7, wherein the composition comprises:
GAG molecular weights in a range of from 66kDa to 99kDa at a concentration of from 5 to 15 mg/mL and comprises GAG molecular weights in the range of from 1OOkDa to 185kDa at a concentration of from 5 to 15 mg/mL;
GAG molecular weights in a range of from 66kDa to 99kDa at a concentration of from 5 to 15 mg/mL and comprises GAG molecular weights in the range of from 301 to 465 kDa at a concentration of from 5 to 15 mg/mL;
GAG molecular weights in a range of from 66kDa to 99kDa at a concentration of from 5 to 15 mg/mL and comprises GAG molecular weights in the range of from 1.01 to 1.80 MDa at a concentration of from 5 to 15 mg/mL;
GAG molecular weights in a range of from 100kDa to 185kDa at a concentration of from 5 to 15 mg/mL and comprises GAG molecular weights in the range of from 301 to 465 kDa at a concentration of from 5 to 15 mg/mL;
GAG molecular weights in a range of from 100kDa to 185kDa at a concentration of from 5 to 15 mg/mL and comprises GAG molecular weights in the range of from 1.01 to 1.80 MDa at a concentration of from 5 to 15 mg/mL; or
GAG molecular weights in a range of from 301 to 465 kDa at a concentration of from 5 to 15 mg/mL and comprises GAG molecular weights in the range of from 1.01 to 1.80 MDa at a concentration of from 5 to 15 mg/mL.

9. The composition according to any preceding claim, wherein the composition is a cell culture medium for eukaryote cells; optionally wherein the cells are ECM-producing cells; optionally wherein the cells are mesenchymal stem cells (MSCs).

10. A cell culture medium comprising the composition according any one of the preceding claims.

11. A method of eukaryote cell culture, the method comprising the step of incubating eukaryote cells in a cell culture medium according to claim 10.

12. A method of enhancing or influencing ECM deposition in eukaryote cell culture, the method comprising the step of incubating eukaryote cells in a cell culture medium according to claim 10.

13. Use of a composition according to any one of claims 1-9, for enhancing or influencing ECM deposition in a cell culture; or as a macromolecular crowding agent in cell culture.

14. A method of manufacturing a cell culture medium, the method comprising adding a first population of GAG molecules to a cell culture medium and adding at least a second population of GAG molecules to the cell culture medium, wherein the first population of GAG molecules has a lower average molecular weight than the second population of GAG molecules.

15. The method according to claim 14, wherein
the first population of GAG molecules has a molecular weight range from 66 kDa to 99 kDa and the second population of GAG molecules has a molecular weight range from 100 kDa to 1800 kDa;
the first population of GAG molecules has a molecular weight range from 66 kDa to 99 kDa and the second population of GAG molecules has a molecular weight range from 100 kDa to 185 kDa;
the first population of GAG molecules has a molecular weight range from 60 kDa to 100 kDa and the second population of GAG molecules has a molecular weight range from 300 kDa to 2000 kDa;
the first population of GAG molecules has a molecular weight range from 60 kDa to 100 kDa and the second population of GAG molecules has a molecular weight range from 300 kDa to 500 kDa;
the first population of GAG molecules has a molecular weight range from 60 kDa to 100 kDa and the second population of GAG molecules has a molecular weight range from 1 to 2 MDa;
the first population of GAG molecules has a molecular weight range from 100 kDa to 200 kDa and the second population of GAG molecules has a molecular weight range from 300 KDa to 2 MDa;
the first population of GAG molecules has a molecular weight range from 100 kDa to 200 kDa and the second population of GAG molecules has a molecular weight range from 300 KDa to 500 KDa;
the first population of GAG molecules has a molecular weight range from 100 kDa to 200 kDa and the second population of GAG molecules has a molecular weight range from 1 to 2 MDa;
the first population of GAG molecules has a molecular weight range from 60 kDa to 500 kDa and the second population of GAG molecules has a molecular weight range from 1 to 2 MDa;
the first population of GAG molecules has a molecular weight range from 100 kDa to 500 kDa and the second population of GAG molecules has a molecular weight range from 1 to 2 MDa; or
the first population of GAG molecules has a molecular weight range from 300 kDa to 500 kDa and the second population of GAG molecules has a molecular weight range from 1 to 2 MDa;
optionally wherein the first and/or second population of GAG molecules are added at a concentration of from 3 to 15 mg/mL.
